# EUROPEAN PATENT APPLICATION

(11) **EP 2 383 814 A1**
(43) Date of publication of application: **02.11.2011**
(21) Application number: 10733515.0
(22) Date of filing: 21.01.2010
(51) Int. Cl.: H01L 51/50, C07D 213/18, C07D 233/58, C08G 61/00, C09K 11/06

(54) **ORGANIC ELECTROLUMINESCENT DEVICE**

(30) Priority: 23.01.2009 JP 2009012559
(71) Applicant: Sumitomo Chemical Company, Limited, Tokyo 104-8260 (JP)
(72) Inventor: KURAMOCHI, Yusuke, Tsukuba-shi Ibaraki 305-0821 (JP); KOBAYASHI, Norifumi, Tsukuba-shi Ibaraki 305-0003 (JP); HIGASHIMURA, Hideyuki, Tsukuba-shi Ibaraki 305-0045 (JP)
(74) Representative: Duckworth, Timothy John
(86) International application number: PCT/JP2010/050699
(87) International publication number: WO 2010/084908

(57) **Abstract**

An organic electroluminescent device in which an organic layer containing a metal coordination compound represented by the following formula (1) is sandwiched between the pair of electrodes composed of an anode and a cathode,

MLₐX_{b} (1)

wherein M is an ion of a metal of Group 11 of the Periodic Table, L is a ligand represented by the formula (2) shown below, X is an anion, and a and b are defined in the specification, 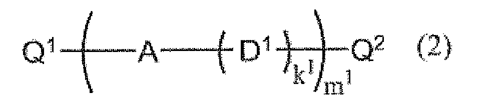 wherein A is a divalent group formed by removing two hydrogen atoms from the formula (3) or (4) shown below, and D¹, k¹, m¹, A, D¹, Q¹, and Q² are defined in the specification, 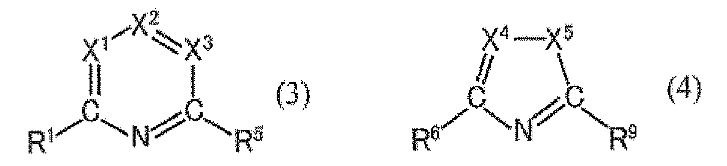 wherein X¹, X², X³, X⁴, X⁵, R¹, R⁵, R⁶, and R⁹ are defined in the specification.

## Description

### Technical Field

The present invention relates to an organic electroluminescent device (hereinafter may be referred to as an organic electroluminescence device or an organic EL device) that uses a metal coordination compound for a luminescent material.

### Background Art

A metal coordination compound, which is a luminescent material using light (phosphorescence) from a triplet excited state, can achieve a luminous efficiency 4 times higher than that of a luminescent material using fluorescence in principle, because the upper limit of its internal quantum efficiency can reach 100%. For a metal coordination compound used in an organic electroluminescent device, a metal coordination compound that uses a platinum group element typified by iridium as a metal is often used.

However, in particular, the amount of iridium resources present in the earth crust is very small, and there is a fear that the resources will be exhausted. Therefore, iridium is expensive.
Accordingly, the use of various metal coordination compounds that use more inexpensive metals advantageous in cost is being contemplated as luminescent materials.

Known examples of such luminescent materials include copper coordination compounds using unsubstituted pyridine (see Non-Patent Document 1).

### Related Art Document

### Non-Patent Document

Non-Patent Document 1: K. R. Kyle et al., Journal of the American Chemical Society 113, 2954-2965 (1991).

### Summary of the Invention

### Problem to be Solved by the Invention

However, the conventional metal coordination compounds described above have insufficient oxygen durability and therefore may suffer deterioration such as decomposition due to exposure to the air (oxygen). Therefore, the conventional metal coordination compounds have a problem in that, when these compounds are used as the luminescent materials of organic EL devices, light is not emitted or their emission lifetime is shortened because, for example, the metal coordination compounds are decomposed by oxygen that has been trapped during the production of the organic EL devices and remains present in the completed organic EL devices.

To solve the above problem, it is an object of the present invention to provide an organic electroluminescent device using, as a luminescent material, a luminescent metal coordination compound that uses a metal of Group 11 of the Periodic Table that is more inexpensive because its reserves and production are larger than those of, for example, iridium, and that has good oxygen durability.

### Means for Solving Problem

The present inventors have made intensive studies on various metal coordination compounds for solving the above problem and found that the above problem can be solved by using, as the luminescent material, a metal coordination compound composed of an inexpensive metal of Group 11 of the Periodic Table and a specific ligand, and thus has completed the present invention.

More specifically, the present invention provides the following [1] to [14].
[1] An organic electroluminescent device in which an organic layer containing a metal coordination compound represented by the following formula (1) is sandwiched between a pair of electrodes composed of an anode and a cathode,

   MLₐX_{b} (1)

   wherein
   M is an ion of a metal of Group 11 of the Periodic Table,
   L is a ligand represented by the formula (2) shown below,
   X is an anion,
   a is a number satisfying 0 < a ≤ 6, and
   b is a number of 0 or more, 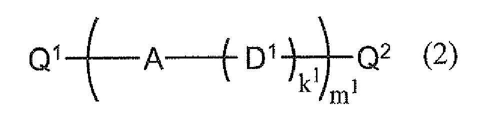
   wherein
   A is a divalent group formed by removing two hydrogen atoms from the formula (3) or the formula (4) shown below,
   D¹ is a divalent group having 1 to 50 carbon atoms,
   k¹ is a number of 0 or more,
   m¹ is a number of 1 or more,
   when at least one of A and D¹ is plurally present, the at least one of A and D¹ that is plurally present may be different from each other, and
   Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, 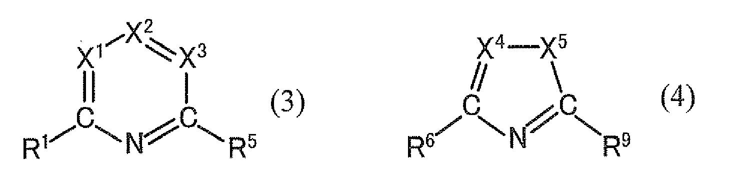
   wherein
   X¹ is a nitrogen atom or a =C(R²)- group, X² is a nitrogen atom or a -C(R³)= group, X³ is a nitrogen atom or a =C(R⁴) - group, X⁴ is a nitrogen atom or a =C(R⁷) - group, X⁵ is a -N(R⁸)- group, N⁻, an oxygen atom, or a sulfur atom, and at least one of X¹, X², and X³ is a =C(R²)- group, a - C(R³) = group, or a =C(R⁴)- group,
   R¹, R², R³, R⁴, R⁵, R⁶, R⁷ and R⁹, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, and R⁸ is a hydrogen atom, a hydrocarbon group optionally having a substituent, or a heterocyclic group optionally having a substituent, provided that at least one of R¹ and R⁵ is a group other than a hydrogen atom and that at least one of R⁶ and R⁹ is a group other than a hydrogen atom, and
   each of a combination of R¹ and R², a combination of R² and R³, a combination of R³ and R⁴, a combination of R⁴ and R⁵, a combination of R⁶ and R⁷, a combination of R⁷ and R⁸, and a combination of R⁸ and R⁹ may together form a ring.
[2] The organic electroluminescent device according to [1], wherein, in the formulae (3) and (4), when R¹ to R⁹ are each a group other than a hydrogen atom, R¹ to R⁹ are each a hydrocarbon group optionally having a substituent.
[3] The organic electroluminescent device according to [1] or [2], wherein, in the formula (2), A is a divalent group of the following formula (5) or (6) with two hydrogen atoms removed therefrom, 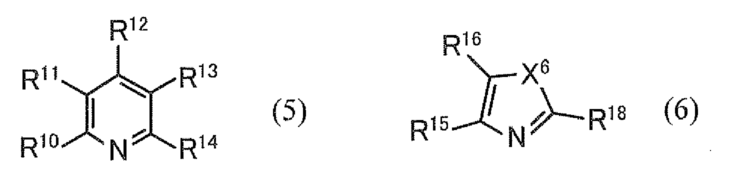 wherein
   X⁶ is a -N (R¹⁷) - group, N⁻, an oxygen atom, or a sulfur atom,
   R¹⁰, R¹¹, R¹², R¹³, R¹⁴, R¹⁵, R¹⁶ and R¹⁸, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, and R¹⁷ is a hydrogen atom, a hydrocarbon group optionally having a substituent, or a heterocyclic group optionally having a substituent, provided that at least one of R¹⁰ and R¹⁴ is a group other than a hydrogen atom and that at least one of R¹⁵ and R¹⁸ is a group other than a hydrogen atom, and
   each of a combination of R¹⁰ and R¹¹, a combination of R¹¹ and R¹², a combination of R¹² and R¹³, a combination of R¹³ and R¹⁴, a combination of R¹⁵ and R¹⁶, a combination of R¹⁶ and R¹⁷, and a combination of R¹⁷ and R¹⁸ may together form a ring.
[4] The organic electroluminescent device according to any one of [1] to [3], wherein, in the formula (2), when A is plurally present, the number of covalent bonds connecting any two coordinating nitrogen atoms by a shortest path is 5 or more.
[5] The organic electroluminescent device according to any one of [1] to [4], wherein, in the formula (2), m¹ is a number from 2 to 10,000.
[6] The organic electroluminescent device according to any one of [1] to [5], wherein, in the metal coordination compound represented by the formula (1), L is a ligand represented by the following formula (7), 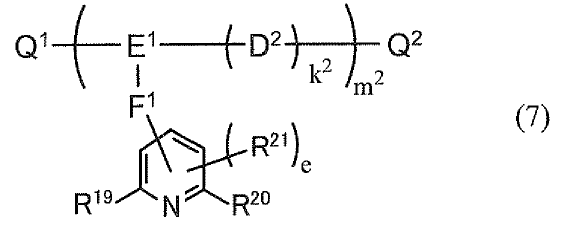 wherein
   Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
   D² is a divalent group having 1 to 50 carbon atoms,
   E¹ is a trivalent group having 1 to 50 carbon atoms,
   F¹ is a direct bond or a divalent group having 1 to 20 carbon atoms,
   m² is a number of 2 or more,
   k² is a number of 0 or more,
   when E¹, F¹, D², R¹⁹, R²⁰, and R²¹ are each plurally present, they each may be different from each other,
   R²¹ is a halogen atom, a cyano group, a nitro group, an acyloxy group optionally having a substituent, a hydrocarbon oxycarbonyl group optionally having a substituent, a carboxylate group, an acylamido group, an imino group, a substituted silyl group, a hydroxy group, an acyl group, a hydrocarbon group optionally having a substituent, a hydrocarbon oxy group optionally having a substituent, a hydrocarbon thio group optionally having a substituent, or a heterocyclic group optionally having a substituent,
   e is a number from 0 to 2,
   R¹⁹ and R²⁰, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, provided that at least one of R¹⁹ and R²⁰ is a group other than a hydrogen atom,
   when a plurality of R²¹s adjoin each other, adjoining R²¹s may together form a ring, and
   when R²¹ and one of R¹⁹ and R²⁰ adjoin each other, R²¹ and the one of R¹⁹ and R²⁰ may together form a ring.
[7] The organic electroluminescent device according to any one of [1] to [5], wherein, in the metal coordination compound represented by the formula (1), L is a ligand represented by the following formula (8), 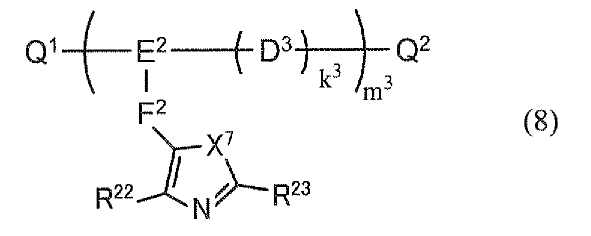 wherein
   Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
   D³ is a divalent group having 1 to 50 carbon atoms,
   E² is a trivalent group having 1 to 50 carbon atoms,
   F² is a direct bond or a divalent group having 1 to 20 carbon atoms,
   m³ is a number of 2 or more,
   k³ is a number of 0 or more,
   when E², F², D³, R²², R²³, and R²⁴ are each plurally present, they each may be different from each other,
   X⁷ is a -N (R²⁴) - group, N⁻, an oxygen atom, or a sulfur atom,
   R²² and R²³, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, and R²⁴ is a hydrogen atom, a hydrocarbon group optionally having a substituent, or a heterocyclic group optionally having a substituent, provided that at least one of R²² and R²³ is a group other than a hydrogen atom.
[8] The organic electroluminescent device according to any one of [1] to [5], wherein, in the metal coordination compound represented by the formula (1), L is a ligand represented by the following formula (9), 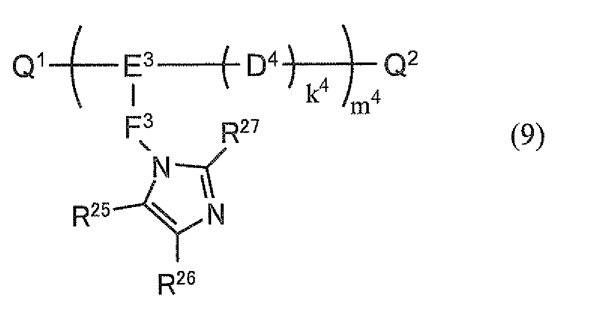 wherein
   Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
   D⁴ is a divalent group having 1 to 50 carbon atoms,
   E³ is a trivalent group having 1 to 50 carbon atoms,
   F³ is a direct bond or a divalent group having 1 to 20 carbon atoms,
   m⁴ is a number of 2 or more,
   k⁴ is a number of 0 or more,
   when E³, F³, D⁴, R²⁵, R²⁶, and R²⁷ are each plurally present, they each may be different from each other,
   R²⁵, R²⁶ and R²⁷, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, provided that at least one of R²⁶ and R²⁷ is a group other than a hydrogen atom, and
   R²⁵ and R²⁶ may together form a ring.
[9] The organic electroluminescent device according to any one of [1] to [8], wherein, in the metal coordination compound represented by the formula (1), L is a ligand represented by the following formula (10), 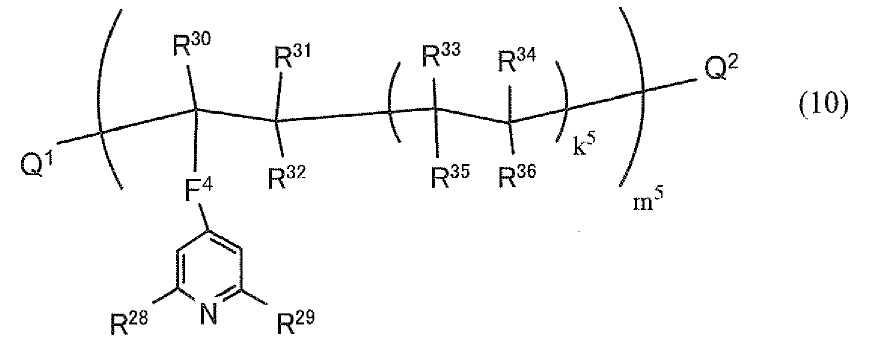 wherein
   Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
   F⁴ is a direct bond or a divalent bond having 1 to 20 carbon atoms,
   m⁵ is a number of 2 or more,
   k⁵ is a number of 0 or more,
   when F⁴, R²⁸, R²⁹, R³⁰, R³¹, R³², R³³, R³⁴, R³⁵, and R³⁶ are each plurally present, they each may be different from each other,
   R²⁸ and R²⁹, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, provided that at least one of R²⁸ and R²⁹ is a group other than a hydrogen atom, and
   R³⁰, R³¹, R³², R³³, R³⁴, R³⁵, and R³⁶, which may be different from each other, are each a hydrogen atom, a halogen atom, a cyano group, an acyloxy group optionally having a substituent, a hydrocarbon oxycarbonyl group optionally having a substituent, an acylamido group, a substituted silyl group, a hydroxy group, an acyl group, a hydrocarbon group optionally having a substituent, a hydrocarbon oxy group optionally having a substituent, or a hydrocarbon thio group optionally having a substituent.
[10]The organic electroluminescent device according to [9], wherein, in the ligand represented by the formula (10), when R²⁸, R²⁹, R³⁰, R³¹, R³², R³³, R³⁴, R³⁵, and R³⁶ are each a carbon atom-containing group, the carbon atom-containing group has 1 to 40 carbon atoms.
[11] The organic electroluminescent device according to claim 1, wherein, in the metal coordination compound represented by the formula (1), L is a ligand selected from among 2,6-dimethylpyridine; poly(4-vinyl-2,6-dimethylpyridine) having a number average molecular weight of at most 13,000; and 1,2,4,5-tetramethylimidazole.
[12] The organic electroluminescent device according to any one of [1] to [11], wherein, in the metal coordination compound represented by the formula (1), M is a monovalent copper ion or a monovalent silver ion.
[13] A ligand represented by the following formula (11), 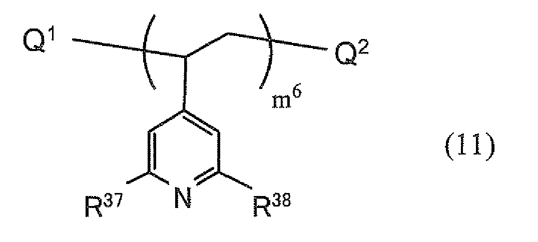 wherein
   Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
   m⁶ is a number from 2 to 10,000, and
   R³⁷ and R³⁸, which may be different from each other and may have a substituent, are each a hydrocarbon group having 1 to 20 carbon atoms.
[14] A metal coordination compound represented by the following formula (12),

   MLₐX_{b} (12)

   wherein
   M is a metal ion,
   L is a ligand represented by the formula (11) shown below,
   X is an anion, a is a number satisfying 0 < a ≤ 6, and
   b is a number of 0 or more, 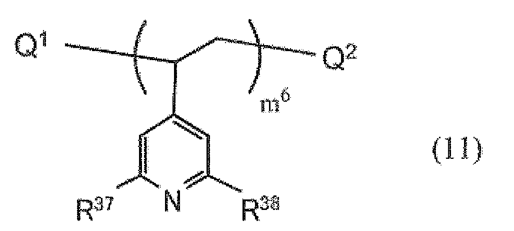
   wherein
   Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
   m⁶ is a number from 2 to 10,000, and
   R³⁷ and R³⁸, which may be different from each other and may have a substituent, are each a hydrocarbon group having 1 to 20 carbon atoms.

### Effects of the Invention

According to the organic electroluminescent device of the present invention, an organic electroluminescent device having good durability particularly against oxygen in the air can be provided at reduced cost.

### Embodiments for Carrying Out the Invention

An organic electroluminescent device of the present invention will be described in detail below.
A luminescent material that can be preferably used for the organic electroluminescent device of the present invention is a metal coordination compound represented by the following formula (1).

MLₐX_{b} (1)

In the formula (1), M is an ion of a metal of Group 11 of the Periodic Table. More specifically, M is a copper ion, a silver ion, or a gold ion, preferably a copper ion or a silver ion, and more preferably a copper ion. The valences of these metal ions are 1 or 2, and preferably 1. L is a ligand represented by formula (2) shown below (the details will be described later).

In the formula (1), X is an anion. Examples of X may include a fluoride ion, a chloride ion, a bromide ion, an iodide ion, a sulfate ion, a nitrate ion, a carbonate ion, an acetate ion, a perchlorate ion, a tetrafluoroborate ion, a hexafluorophosphate ion, a hexafluoro antimony ion, a hexafluoro arsenic ion, a methanesulfonate ion, a trifluoromethanesulfonate ion, a trifluoroacetate ion, a benzenesulfonate ion, a p-toluenesulfonate ion, a dodecylbenzenesulfonate ion, a tetraphenylborate ion, a tetrakis(pentafluorophenyl)borate ion, and high-molecular compounds containing any suitable repeating units and having the structures of any of the above ions. X is preferably a fluoride ion, a chloride ion, a bromide ion, an iodide ion, a sulfate ion, a nitrate ion, a perchlorate ion, a tetrafluoroborate ion, a hexafluorophosphate ion, a tetraphenylborate ion, or a tetrakis(pentafluorophenyl)borate ion, and more preferably a chloride ion, a bromide ion, or an iodide ion. When L is a negatively charged ionic ligand, the metal coordination compound may not include any anions. X may be included singly or in a combination of two or more of these ions in a molecule.

In the formula (1), a and b are determined by the number of coordinating atoms in the ligand and the feed ratio for synthesis. a is a number satisfying 0 < a ≤ 6, preferably a number satisfying 0 < at ≤ 3, more preferably a number satisfying 0 < a ≤ 2, and still more preferably a number satisfying 0 < a ≤ 1.
b is a number of 0 or more, preferably a number satisfying 0 ≤ b ≤ 2, and more preferably a number satisfying 0 < a ≤ 1.

In the formula (1), L is a ligand represented by the following formula (2).

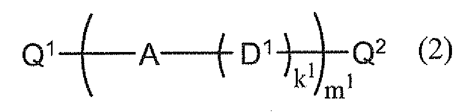

In the formula (2), A is a divalent group formed by removing two hydrogen atoms from a compound defined by the following formula (3) or (4).

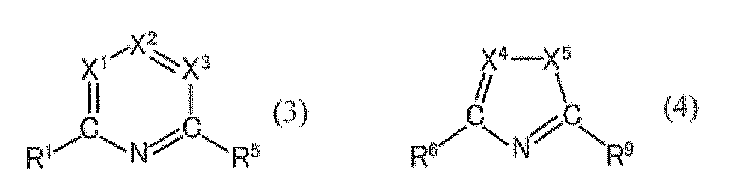

In the formulae (3) and (4), X¹ is a nitrogen atom or a =C(R²) - group, X² is a nitrogen atom or a -C(R³)= group, X³ is a nitrogen atom or a =C(R⁴)- group, X⁴ is a nitrogen atom or a -C(R⁷)- group, X⁵ is a -N(R⁸)- group, N⁻, an oxygen atom, or a sulfur atom, and at least one of X¹, X², and X³ is a =C(R²)- group, a -C(R³)= group, or a =C(R⁴)-group. R¹, R², R³, R⁴, R⁵, R⁶, R⁷, and R⁹, which may be different from each other, are each a hydrogen atom, a halogen atom, a cyano group, a hydroxy group, a nitro group, or a carbon atom-containing group. The carbon atom-containing group has 1 to 40 carbon atoms and is an acyloxy group (an RCOO- group: R is a hydrocarbon group) optionally having a substituent, a hydrocarbon oxycarbonyl group (an ROCO- group: R is a hydrocarbon group) optionally having a substituent, a carboxylate group, an acylamido group (an RCONH- group: R is a hydrocarbon group), an imino group (an =NH group), a substituted silyl group, an acyl group, a hydrocarbon group optionally having a substituent, a hydrocarbon oxy group (an RO- group: R is a hydrocarbon group) optionally having a substituent, a hydrocarbon thio group (an RS- group: R is a hydrocarbon group), or a heterocyclic group optionally having a substituent. R⁸ is a hydrogen atom, a hydrocarbon group optionally having a substituent, or a heterocyclic group optionally having a substituent. When R, R¹, R², R³, R⁴, R⁵, R⁶, R⁷, R⁸, and R⁹ are each a carbon atom-containing group, the number of carbon atoms is preferably 1 to 30, more preferably 1 to 20, still more preferably 1 to 10, and particularly preferably 1 to 6. In particular, when any of R, R¹, R², R³, R⁴, R⁵, R⁶, R⁷, R⁸, and R⁹ is a group having an aromatic ring, the number of carbon atoms is preferably 5 to 30, more preferably 6 to 20, and still more preferably 6 to 10.
However, at least one of R¹ and R⁵ is a group other than a hydrogen atom, and at least one of R⁶ and R⁹ is a group other than a hydrogen atom.
Each of a combination of R¹ and R², a combination of R² and R³, a combination of R³ and R⁴, a combination of R⁴ and R⁵, a combination of R⁶ and R⁷, a combination of R⁷ and R⁸, and a combination of R⁸ and R⁹ may together form a ring.

Examples of the hydrocarbon group in R¹ to R⁹ may include a methyl group, an ethyl group, a 1-propyl group, a 2-propyl group, a 1-butyl group, a 2-butyl group, a sec-butyl group, a tert-butyl group, a pentyl group, a hexyl group, an octyl group, a decyl group, a dodecyl group, a 2-ethylhexyl group, a 3,7-dimethyloctyl group, a cyclopropyl group, a cyclopentyl group, a cyclohexyl group, a 1-adamantyl group, a 2-adamantyl group, a norbornyl group, an ammonium ethyl group, a benzyl group, an α,α-dimethylbenzyl group, a 1-phenethyl group, a 2-phenethyl group, a vinyl group, a propenyl group, a butenyl group, an oleyl group, an eicosapentaenyl group, a docosahexaenyl group, a 2,2-diphenylvinyl group, a 1,2,2-triphenylvinyl group, a 2-phenyl-2-propenyl group, a phenyl group, a 2-tolyl group, a 4-tolyl group, a 4-trifluoromethylphenyl group, a 4-methoxyphenyl group, a 4-cyanophenyl group, a 2-biphenylyl group, a 3-biphenylyl group, a 4-biphenylyl group, a terphenylyl group, a 3,5-diphenylphenyl group, a 3,4-diphenylphenyl group, a pentaphenylphenyl group, a 4-(2, 2-diphenylvinyl)phenyl group, a 4-(1,2,2-triphenylvinyl)phenyl group, a fluorenyl group, a 1-naphthyl group, a 2-naphthyl group, a 9-anthryl group, a 2-anthryl group, a 9-phenanthryl group, a 1-pyrenyl group, a chrysenyl group, a naphthacenyl group, and a coronyl group. The hydrocarbon group in R¹ to R⁹ is preferably a methyl group, an ethyl group, a 1-propyl group, a 2-propyl group, a 1-butyl group, a 2-butyl group, a sec-butyl group, a pentyl group, a hexyl group, an octyl group, a decyl group, a dodecyl group, a 2-ethylhexyl group, a 3,7-dimethyloctyl group, a benzyl group, an α, α-dimethylbenzyl group, a 1-phenethyl group, a 2-phenethyl group, a vinyl group, a propenyl group, a butenyl group, an oleyl group, an eicosapentaenyl group, a docosahexaenyl group, a 2,2-diphenylvinyl group, a 1,2,2-triphenylvinyl group, a 2-phenyl-2-propenyl group, a phenyl group, a 2-tolyl group, a 4-tolyl group, a 4-trifluoromethylphenyl group, a 4-methoxyphenyl group, a 4-cyanophenyl group, a 2-biphenylyl group, a 3-biphenylyl group, a 4-biphenylyl group, a terphenylyl group, a 3,5-diphenylphenyl group, a 3,4-diphenylphenyl group, a pentaphenylphenyl group, a 4-(2,2-diphenylvinyl)phenyl group, a 4-(1,2,2-triphenylvinyl)phenyl group, a fluorenyl group, a 1-naphthyl group, a 2-naphthyl group, a 9-anthryl group, a 2-anthryl group, or a 9-phenanthryl group; more preferably a methyl group, an ethyl group, a 1-propyl group, a 2-propyl group, a 1-butyl group, a 2-butyl group, a sec-butyl group, a pentyl group, a hexyl group, an octyl group, a decyl group, a dodecyl group, a 2-ethylhexyl group, a 3,7-dimethyloctyl group, a benzyl group, a phenyl group, a 2-biphenylyl group, a 3-biphenylyl group, a 4-biphenylyl group, or a terphenylyl group; and still more preferably a methyl group, an ethyl group, a 1-propyl group, a 2-propyl group, a 1-butyl group, a 2-butyl group, a sec-butyl group, a pentyl group, a hexyl group, an octyl group, a decyl group, a dodecyl group, or a 2-ethylhexyl group.

The heterocyclic group as used herein is an atomic group (a residue) formed by removing one hydrogen atom from a heterocyclic compound. Examples of the heterocyclic group may include a furyl group, a thienyl group, a pyrrolyl group, an imidazolyl group, an oxazolyl group, a thiazolyl group, and a pyridyl group. Preferably, the heterocyclic group is a furyl group, a thienyl group, or a pyrrolyl group.

Examples of the halogen atom described above may include a fluorine atom, a chlorine atom, a bromine atom, and an iodine atom. Preferably, the halogen atom is a fluorine atom, a chlorine atom, or a bromine atom.

Examples of the acyloxy group described above may include an acetoxy group, a propionyloxy group, a benzoyloxy group, a naphthylcarbonyloxy group, and a 2-ethylhexylcarbonyloxy group. Preferably, the acyloxy group is an acetoxy group or a benzoyloxy group.

Examples of the hydrocarbon oxycarbonyl group described above may include a methoxycarbonyl group, an ethoxycarbonyl group, a propoxycarbonyl group, a benzoxycarbonyl group, a naphthoxycarbonyl group, and a 2-ethylhexoxycarbonyl group. Preferably, the hydrocarbon oxycarbonyl group is a methoxycarbonyl group or an ethoxycarbonyl group.

The carboxylate group described above is a group represented by -C(=O)O⁻ and may have a cation as a counter ion. Specific examples of the cation may include Li⁺, Na⁺, K⁺, Rb⁺, Cs⁺, Mg²⁺, Ca²⁺, Sr²⁺, and Ba²⁺. The cation is preferably Li⁺, Na⁺, K⁺, or Rb⁺, and more preferably Na⁺ or K⁺.

Examples of the acylamido group described above may include a formamido group, an acetamido group, a propionamido group, a butyroamido group, a benzamido group, a trifluoroacetamido group, a pentafluorobenzamido group, a diformamido group, a diacetamido group, a dipropionamido group, a dibutyroamido group, a dibenzamido group, a ditrifluoroacetamido group, and a dipentafluorobenzamido group. Preferably, the acylamido group is a formamido group, an acetamido group, a propionamido group, a butyroamido group, or a benzamido group.

Examples of the imino group described above may include an N-succinimido group, an N-phthalimido group, and a benzophenoneimido group. Preferably, the imino group is an N-phthalimido group.

The substituted silyl group described above includes a silyl group substituted with one, two, or three groups selected from the group consisting of an alkyl group, an aryl group, and an arylalkyl group, and the alkyl, aryl, or arylalkyl group may further have a substituent.
Examples of the substituted silyl group may include a trimethylsilyl group, a triethylsilyl group, a tripropylsilyl group, a tri-i-propylsilyl group, a dimethyl-i-propylsilyl group, a diethyl-i-propylsilyl group, a t-butyldimethylsilyl group, a pentyldimethylsilyl group,
a hexyldimethylsilyl group, a heptyldimethylsilyl group, an octyldimethylsilyl group, a 2-ethylhexyl-dimethylsilyl group, a nonyldimethylsilyl group, a decyldimethylsilyl group, a 3,7-dimethyloctyl-dimethylsilyl group, a lauryldimethylsilyl group, a triphenylsilyl group, a tri-p-xylylsilyl group, a tribenzylsilyl group, a diphenylmethylsilyl group, a t-butyldiphenylsilyl group, and a dimethylphenylsilyl group. The substituted silyl group is preferably a trimethylsilyl group, a triethylsilyl group, or a tripropylsilyl group, and more preferably a trimethylsilyl group.

Examples of the acyl group described above may include an acetyl group, a propionyl group, a butyryl group, an isobutyryl group, a pivaloyl group, a benzoyl group, a trifluoroacetyl group, and a pentafluorobenzoyl group. Preferably, the acyl group is an acetyl group, a propionyl group, or a benzoyl group.

Examples of the hydrocarbon oxy group described above may include a methoxy group, an ethoxy group, a 1-propyloxy group, a 2-propyloxy group, a 1-butyloxy group, a 2-butyloxy group, a sec-butyloxy group, a tert-butyloxy group, a pentyloxy group, a hexyloxy group, an octyloxy group, a decyloxy group, a dodecyloxy group, a 2-ethylhexyloxy group, a 3, 7-dimethyloctyloxy group, a cyclopropyloxy group, a cyclopentyloxy group, a cyclohexyloxy group, a 1-adamantyloxy group, a 2-adamantyloxy group, a norbornyloxy group, an ammonium ethoxy group, a trifluoromethoxy group, a benzyloxy group, an α, α-dimethylbenzyioxy group, a 2-phenethyloxy group, a 1-phenethyloxy group, a phenoxy group, an alkoxyphenoxy group, an alkylphenoxy group, a 1-naphthyloxy group, a 2-naphthyloxy group, and a pentafluorophenyloxy group. The hydrocarbon oxy group is preferably a methoxy group, an ethoxy group, a 1-propyloxy group, a 2-propyloxy group, a 1-butyloxy group, a 2-butyloxy group, a sec-butyloxy group, a tert-butyloxy group, a pentyloxy group, a hexyloxy group, an octyloxy group, a decyloxy group, a dodecyloxy group, a 2-ethylhexyloxy group, or a 3,7-dimethyloctyloxy group, and more preferably a methoxy group or an ethoxy group.

Examples of the hydrocarbon thio group described above may include a methylthio group, an ethylthio group, a 1-propylthio group, a 2-propylthio group, a 1-butylthio group, a 2-butylthio group, a sec-butylthio group, a tert-butylthio group, a pentylthio group, a hexylthio group, an octylthio group, a decylthio group, a dodecylthio group, a 2-ethylhexylthio group, a 3,7-dimethyloctylthio group, a cyclopropylthio group, a cyclopentylthio group, a cyclohexylthio group, a 1-adamantylthio group, a 2-adamantylthio group, a norbornylthio group, an ammonium ethylthio group, a trifluoromethylthio group, a benzylthio group, an α,α-dimethylbenzylthio group, a 2-phenethylthio group, a 1-phenethylthio group, a phenylthio group, an alkoxyphenylthio group, an alkylphenylthio group, a 1-naphthylthio group, a 2-naphthylthio group, and a pentafluorophenylthio group. The hydrocarbon thio group is preferably a methylthio group, an ethylthio group, a 1-propylthio group, a 2-propylthio group, a 1-butylthio group, a 2-butylthio group, a sec-butylthio group, a pentylthio group, a hexylthio group, an octylthio group, a decylthio group, a dodecylthio group, a 2-ethylhexylthio group, or a 3,7-dimethyloctylthio group, and more preferably a methylthio group or an ethylthio group.

The "substituent" as used herein refers to a halogen atom, a cyano group, a nitro group, an acyloxy group, a hydrocarbon oxycarbonyl group, a carboxylate group, an acylamido group, an imino group, a hydroxy group, an acyl group, a hydrocarbon group, a hydrocarbon oxy group, a hydrocarbon thio group, or a heterocyclic group, when the "substituent" is bonded to a carbon atom. Specific examples and preferred examples of each of the above groups are the same as those of the corresponding group described above for the compounds defined by the formulae (3) and (4).
As for the acyloxy group, hydrocarbon oxycarbonyl group, hydrocarbon group, hydrocarbon oxy group, and hydrocarbon thio group among the above "substituents" bonded to carbon atoms, a hydrogen atom in each group may be further substituted with a halogen atom, a cyano group, a nitro group, an acyloxy group, a hydrocarbon oxycarbonyl group, a carboxylate group, an acylamido group, an imino group, a hydroxy group, an acyl group, a hydrocarbon group, a hydrocarbon oxy group, a hydrocarbon thio group, or a heterocyclic group.
Preferred examples of the "substituent" bonded to a carbon atom may include a halogen atom, a cyano group, an acyloxy group, a hydrocarbon oxycarbonyl group, an acylamido group, an acyl group, a hydrocarbon group, a hydrocarbon oxy group, and a hydrocarbon thio group. The "substituent" bonded to a carbon atom is more preferably a halogen atom, a hydrocarbon oxycarbonyl group, an acylamido group, an acyl group, a hydrocarbon group, or a hydrocarbon oxy group, still more preferably a halogen atom, a hydrocarbon group, or a hydrocarbon oxy group, and particularly preferably a hydrocarbon group.

The "substituent" as used herein refers to a hydrocarbon oxycarbonyl group, an acyl group, or a hydrocarbon group, when the "substituent" is bonded to a nitrogen atom. Specific examples and preferred examples of each of the above groups are the same as those of the corresponding group described above for the compounds defined by the formulae (3) and (4).
As for the hydrocarbon oxycarbonyl group and hydrocarbon group among the "substituents" bonded to nitrogen atoms may be further substituted with a halogen atom, a cyano group, a nitro group, an acyloxy group, a hydrocarbon oxycarbonyl group, a carboxylate group, an acylamido group, an imino group, a hydroxy group, an acyl group, a hydrocarbon group, a hydrocarbon oxy group, a hydrocarbon thio group, or a heterocyclic group. Preferred examples of the "substituent" bonded to a nitrogen atom may include a hydrocarbon group.

The substituent that may be included in the "substituent" bonded to a carbon atom or the "substituent" bonded to a nitrogen atom is preferably a halogen atom, a hydrocarbon group, or a hydrocarbon oxy group, and more preferably a hydrocarbon group.

In the formulae (3) and (4), X¹ is preferably a =C(R²)- group, X² is preferably a -C(R³)= group, X³ is preferably a =C(R⁴)- group, and X⁴ is preferably a =C(R⁷)-group. More preferably, these R², R³, R⁴, and R⁷ are each a hydrogen atom, a hydrocarbon group optionally having a substituent, a hydrocarbon oxy group optionally having a substituent, or a hydrocarbon thio group optionally having a substituent. Still more preferably, these R², R³, R⁴, and R⁷ are each a hydrogen atom or a hydrocarbon group optionally having a substituent.

In the formula (4), X⁵ is preferably a -N (R⁸) - group, a sulfur atom, or an oxygen atom, and more preferably a -N (R⁸) - group. Still more preferably, R⁸ is a hydrogen atom or a hydrocarbon group optionally having a substituent.

In the formula (3), at least one of R¹ and R⁵ is a group other than a hydrogen atom. Preferably, both R¹ and R⁵ are each a group other than a hydrogen atom. In the formula (4), at least one of R⁶ and R⁹ is a group other than a hydrogen atom. Preferably, both R⁶ and R⁹ are each a group other than a hydrogen atom. In the formulae (3) and (4), R¹, R⁵, R⁶ and R⁹ are each preferably a hydrocarbon group optionally having a substituent, a hydrocarbon oxy group optionally having a substituent, or a hydrocarbon thio group optionally having a substituent, and more preferably a hydrocarbon group optionally having a substituent.

In the ligand represented by the formula (2), A is preferably a divalent group of the following formula (5) or (6) with two hydrogen atoms removed therefrom.

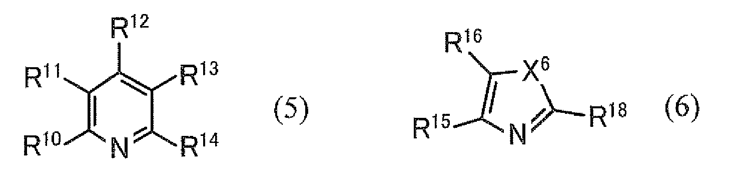

In the above formulae, X⁶ is a -N (R¹⁷) - group, N⁻, an oxygen atom, or a sulfur atom.
R¹⁰, R¹¹, R¹², R¹³, R¹⁴, R¹⁵, R¹⁶, and R¹⁸, which may be different from each other, are each a hydrogen atom, a halogen atom, a cyano group, a hydroxy group, a nitro group, or a carbon atom-containing group. The carbon atom-containing group has 1 to 40 carbon atoms and is an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent. R¹⁷ is a hydrogen atom, a hydrocarbon group optionally having a substituent, or a heterocyclic group optionally having a substituent. However, at least one of R¹⁰ and R¹⁴ is a group other than a hydrogen atom, and at least one of R¹⁵ and R¹⁸ is a group other than a hydrogen atom.
At least one of a combination of R¹⁰ and R¹¹, a combination of R¹¹ and R¹², a combination of R¹² and R¹³, a combination of R¹³ and R¹⁴, a combination of R¹⁵ and R¹⁶, a combination of R¹⁶ and R¹⁷, and a combination of R¹⁷ and R¹⁸ may together form a ring.

Examples of A may include a divalent group formed by removing two hydrogen atoms from any one of compounds A1 to A52 shown below. Ph represents a phenyl group; ^{t}Bu represents a tert-butyl group; Me represents a methyl group; Et represents an ethyl group; and ⁱPr represents an isopropyl group.

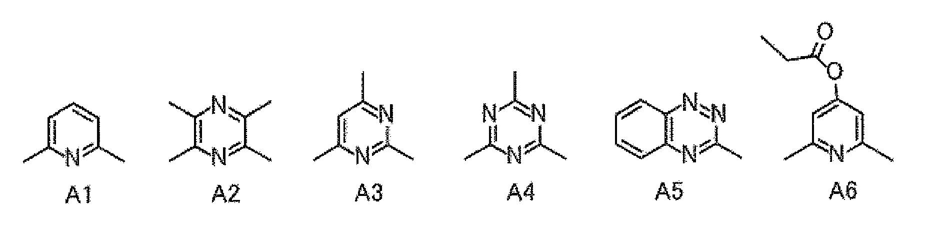

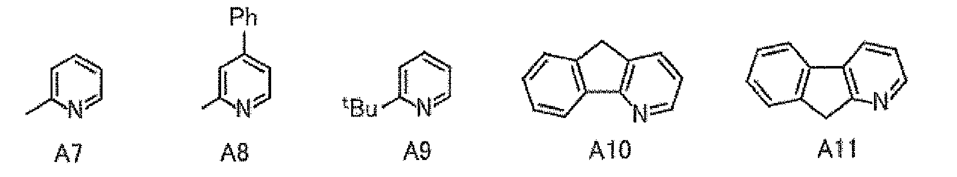

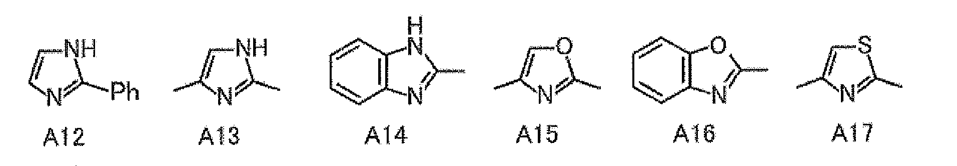

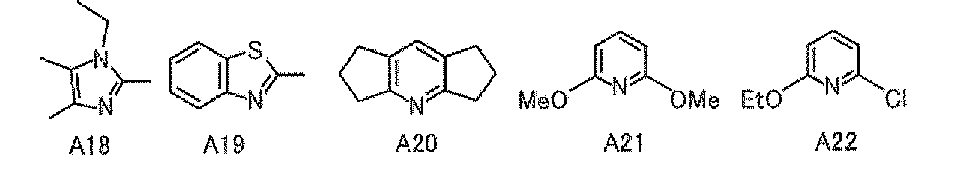

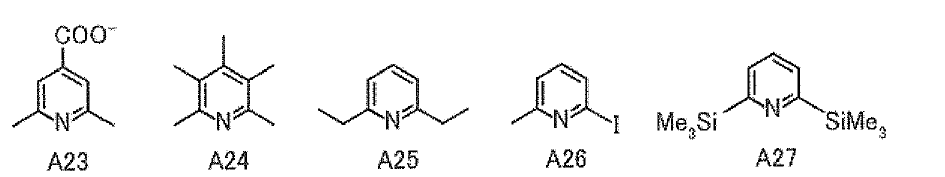

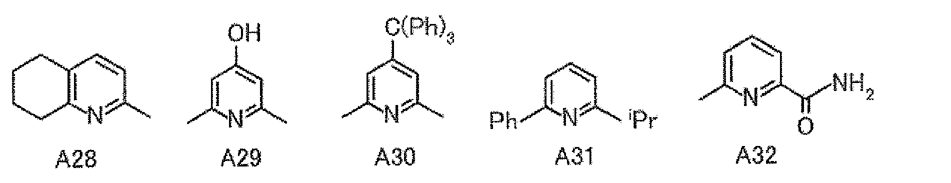

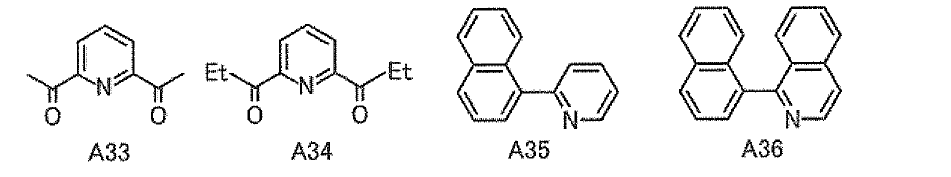

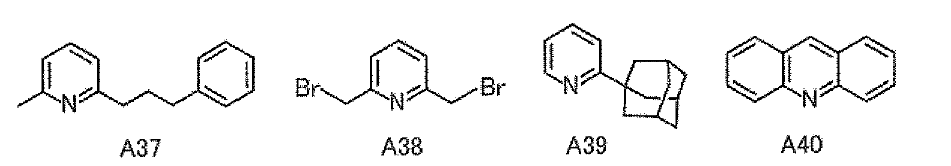

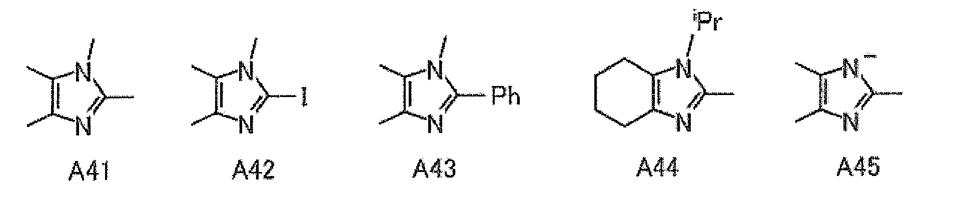

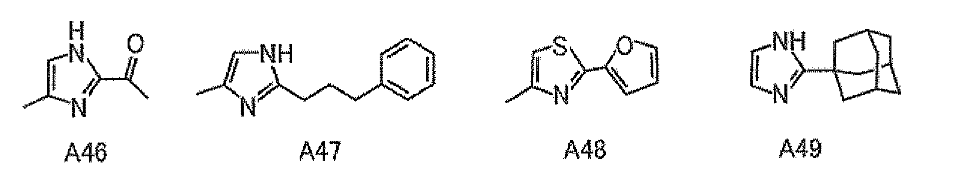

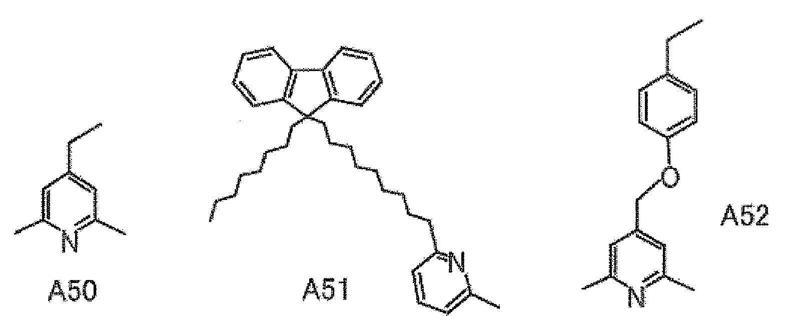

In the formula (2), D¹ is a divalent group having 1 to 50 carbon atoms and is an alkanediyl group optionally having a substituent, an alkenediyl group optionally having a substituent, a divalent aromatic group optionally having a substituent, or a group represented by any one of formulae (1)' to (10)' shown below. D¹ has preferably 1 to 40 carbon atoms, more preferably 1 to 30 carbon atoms, still more preferably 1 to 20 carbon atoms, and particularly preferably 1 to 10 carbon atoms. Particularly, when D¹ is a group having an aromatic ring, D¹ has preferably 5 to 40 carbon atoms, more preferably 5 to 30 carbon atoms, still more preferably 6 to 20 carbon atoms, and particularly preferably 6 to 10 carbon atoms.

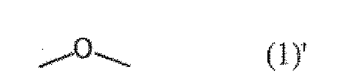

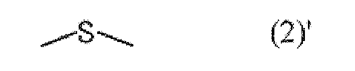

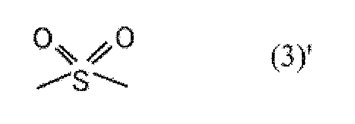

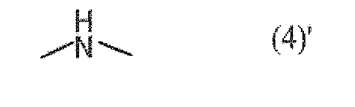

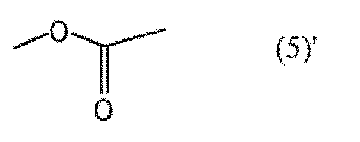

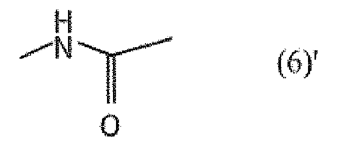

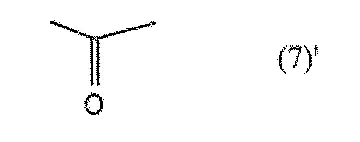

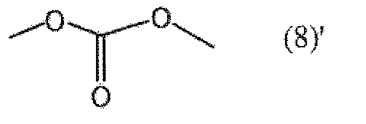

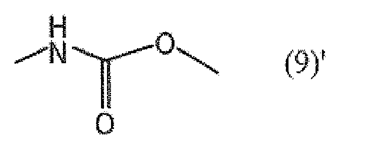

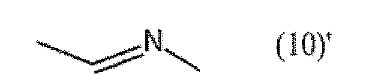

Examples of the alkanediyl group described above may include a methylene group, an ethylene group, a propylene group, a butylene group, a pentylene group, a hexylene group, an octylene group, a nonylene group, a dodecylene group, a cyclopropylene group, a cyclobutylene group, a cyclopentylene group, and a cyclohexylene group. The alkanediyl group is preferably a methylene group, an ethylene group, or a propylene group, and more preferably an ethylene group.

Examples of the alkenediyl group described above may include an ethenylene group, a propenylene group, a 3-butenylene group, a 2-pentenylene group, a 2-hexenylene group, a 2-nonenylene group, a 2-dodecenylene ethenylene group, a propenylene group, and a butenylene group. The alkenediyl group is preferably an ethenylene group or a propenylene group, and more preferably an ethenylene group.

Examples of the divalent aromatic group may include a group formed by removing two hydrogen atoms from any one of rings represented by Ar-1 to Ar-46 below.

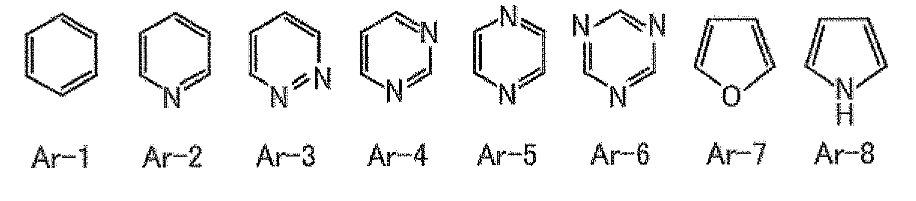

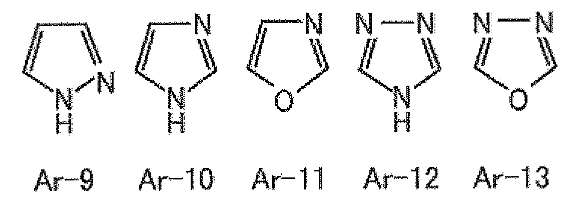

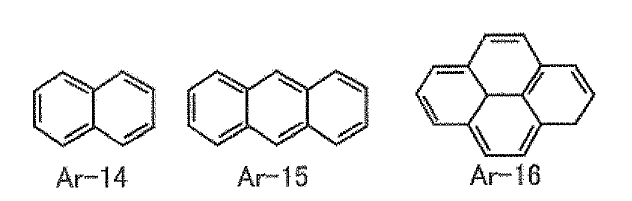

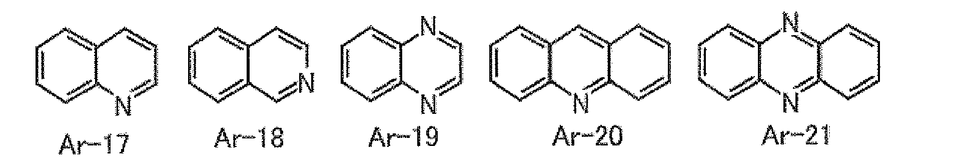

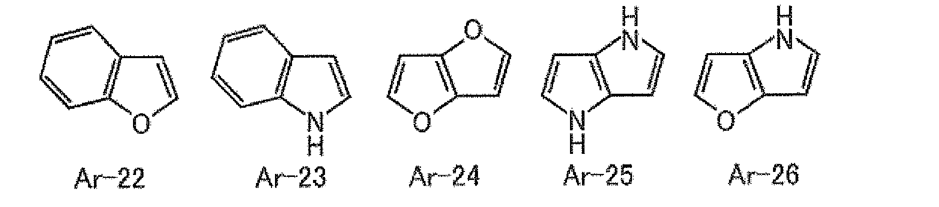

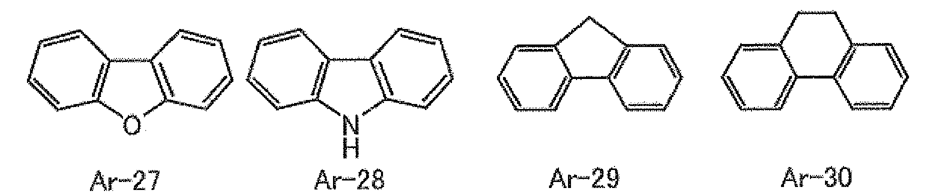

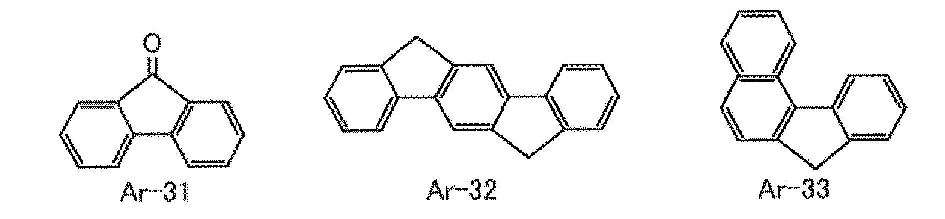

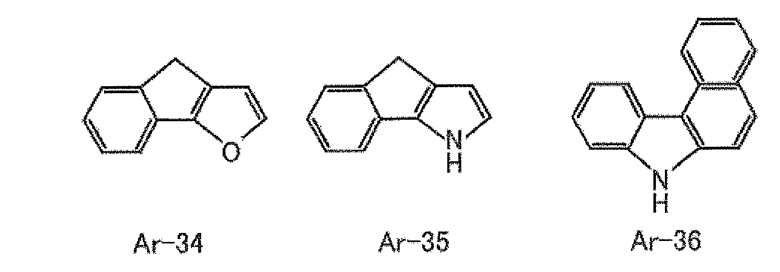

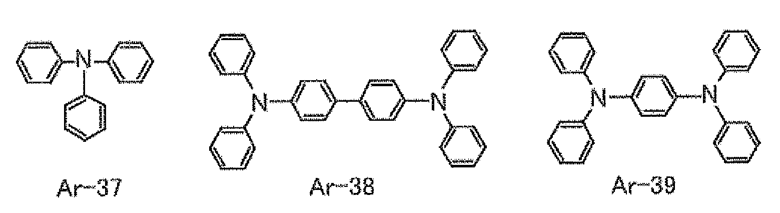

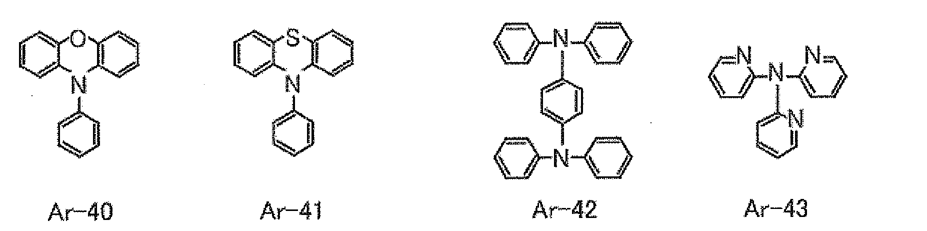

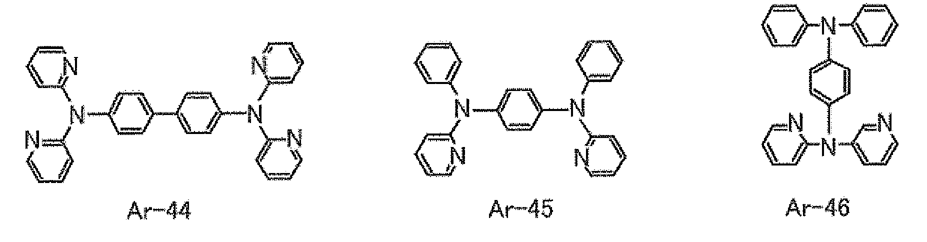

D¹ may also be a group formed by any combination of the alkanediyl groups, alkenediyl groups, divalent aromatic groups, and groups represented by any one of the formulae (1)' to (10)'. Examples of such a group may include a group having the structure represented by any one of the following D-1 to D-8.

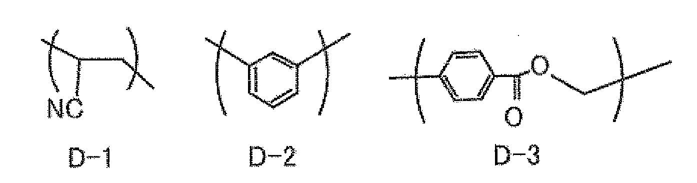

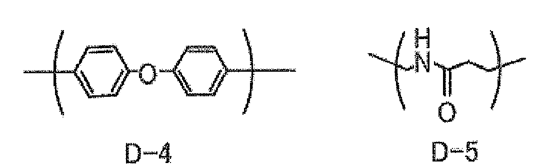

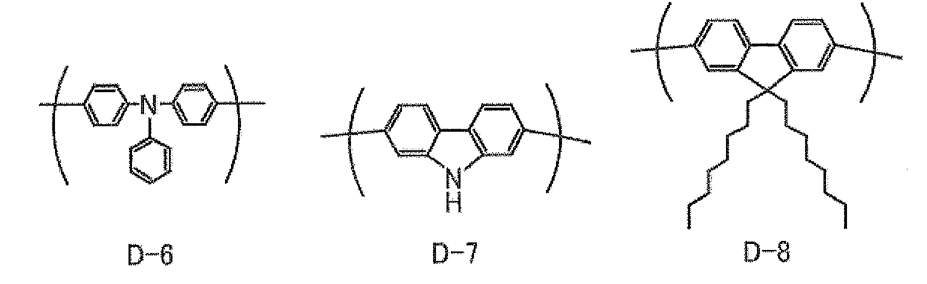

In the formula (2), Q¹ and Q², which may be different from each other, are each a hydrogen atom, a halogen atom, a cyano group, a hydroxy group, a nitro group, or a carbon atom-containing group. The carbon atom-containing group has 1 to 40 carbon atoms and is an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent. Q¹ and Q² are each preferably a hydrogen atom, a hydrocarbon group optionally having a substituent, a hydrocarbon oxy group optionally having a substituent, or a hydrocarbon thio group optionally having a substituent, more preferably a hydrogen atom or a hydrocarbon group optionally having a substituent, and still more preferably a hydrogen atom. Specific examples and preferred examples of each of the above groups are the same as those of the corresponding group described above for the compounds defined by the formulae (3) and (4).
When Q¹ and Q² are each a carbon atom-containing group, the group has preferably 1 to 20 carbon atoms, more preferably 1 to 10 carbon atoms, and still more preferably 1 to 6 carbon atoms. Particularly, when Q¹ and Q² are each a group having an aromatic ring, the group has 5 to 20 carbon atoms, and more preferably 6 to 10 carbon atoms.

In the formula (2), k¹ is a number of 0 or more, preferably 0 to 1,000, more preferably 0 to 100, still more preferably 0 to 20, and particularly preferably 0 to 10.

In the formula (2), m¹ is a number of 1, or 2 or more. When m¹ is 2 or more, it is preferably a number from 2 to 10,000, more preferably a number from 2 to 1,000, still more preferably a number from 5 to 500, and particularly preferably a number from 10 to 500. When m¹ has a distribution, m¹ represents the average value.

When there are a plurality of As or D¹s, they each may be different from each other.

When there are a plurality of As, from the viewpoint of luminous efficiency, the number of covalent bonds connecting nitrogen atoms coordinating to the M described above (i.e., the number of covalent bonds connecting two coordinating nitrogen atoms by the shortest path) is preferably 5 or more, more preferably 5 or more and 100 or less, still more preferably 5 or more and 30 or less, and particularly preferably 5 or more and 10 or less.
In the ligand represented by the formula (2), when there are three or more coordinating nitrogen atoms, the number of covalent bonds connecting any two of these coordinating nitrogen atoms by the shortest path is 5 or more.

Preferably, L in the formula (1) has the structure represented by the following formula (7), (8), or (9).

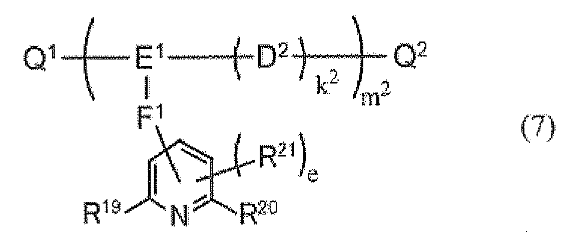

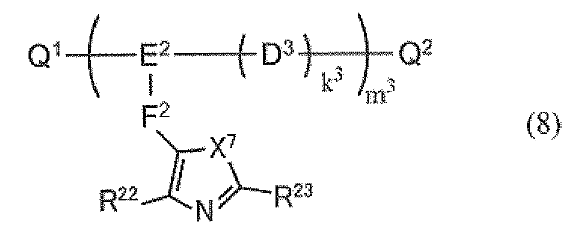

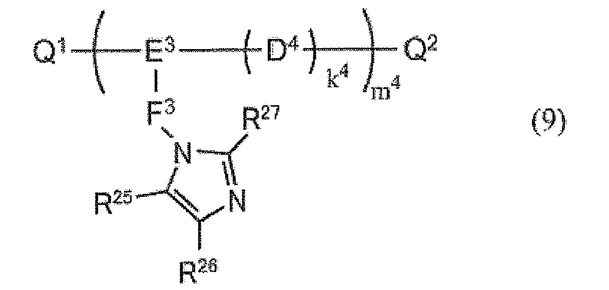

Each of D² in the formula (7), D³ in the formula (8), and D⁴ in the formula (9) is the same as D¹ defined in the formula (2). Specific examples and preferred examples of D², D³, and D⁴ are also the same as those of D¹ defined in the formula (2).

E¹ in the formula (7), E² in the formula (8), and E³ in the formula (9) are each a nitrogen atom or a trivalent group having 1 to 50 carbon atoms. Examples of the trivalent group having 1 to 50 carbon atoms may include an alkanetriyl group optionally having a substituent, an alkenetriyl group optionally having a substituent, and a trivalent aromatic group optionally having a substituent. The number of carbon atoms is preferably 1 to 40, more preferably 1 to 30, still more preferably 1 to 20, and particularly preferably 1 to 10.

Examples of the alkanetriyl group described above may include a group formed by removing three hydrogen atoms from methane, ethane, propane, butane, pentane, hexane, octane, nonane, dodecane, cyclopropane, cyclobutane, cyclopentane, or cyclohexane. The alkanetriyl group is preferably a group formed by removing three hydrogen atoms from methane, ethane, or propane, and more preferably a group formed by removing three hydrogen atoms from ethane.

Examples of the alkenetriyl group described above may include a group formed by removing three hydrogen atoms from ethene, propene, 3-butene, 2-pentene, 2-hexane, or 2-nonene. The alkenetriyl group is preferably a group formed by removing three hydrogen atoms from ethene or propene, and more preferably a group formed by removing three hydrogen atoms from ethene.

Examples of the trivalent aromatic group described above may include a group formed by removing three hydrogen atoms from a ring represented by any one of Ar-1 to Ar-46 described above.

F¹ in the formula (7), F² in the formula (8), and F³ in the formula (9) are each a direct bond or a divalent group having 1 to 20 carbon atoms. The divalent group is an alkanediyl group optionally having a substituent, an alkenediyl group optionally having a substituent, a divalent aromatic group optionally having a substituent, or a group represented by any one of the above formulae (1)' to (10)'.

Examples of the alkanediyl group described above may include a methylene group, an ethylene group, a propylene group, a butylene group, a pentylene group, a hexylene group, an octylene group, a nonylene group, a dodecylene group, a cyclopropylene group, a cyclobutylene group, a cyclopentylene group, and a cyclohexylene group. The alkanediyl group is preferably a methylene group, an ethylene group, or a propylene group, and more preferably an ethylene group.

Examples of the alkenediyl group described above may include an ethenylene group, a propenylene group, a 3-butenylene group, a 2-pentenylene group, a 2-hexenylene group, a 2-nonenylene group, a 2-dodecenylene ethenylene group, a propenylene group, and a butenylene group. The alkenediyl group is preferably an ethenylene group or a propenylene group, and more preferably an ethenylene group.

Examples of the divalent aromatic group may include a group formed by removing two hydrogen atoms from a ring represented by any one of the following Ar-47 to Ar-59.

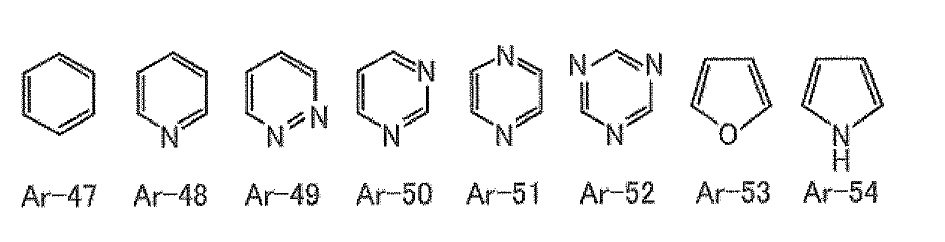

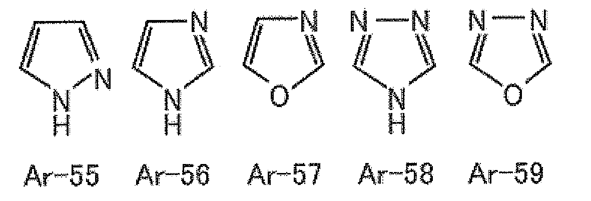

F¹ in the formula (7), F² in the formula (8), and F³ in the formula (9) may be each a group composed of any combination of the above-described alkanediyl groups, alkenediyl groups, divalent aromatic groups, and formulae (1)' to (10)'. Examples of such a group may include a group having the structure represented by any one of the following F-1 to F-5.

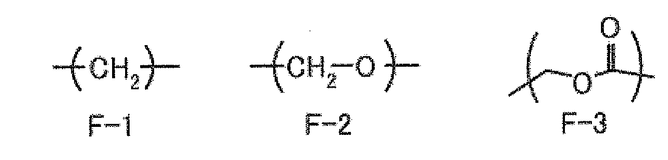

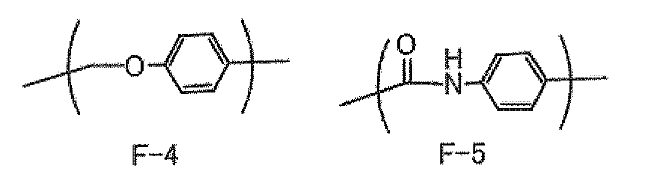

m² in the formula (7), m³ in the formula (8), and m⁴ in the formula (9) are each a number of 2 or more, preferably 2 to 10,000, more preferably 2 to 1,000, and still more preferably 5 to 500.

k² in the formula (7), k³ in the formula (8), and k⁴ in the formula (9) are each a number of 0 or more, preferably 0 to 1, 000, more preferably 0 to 100, still more preferably 0 to 20, and particularly preferably 0 to 10.

When E¹, E², E³, F¹, F², F³, D², D³, D⁴, R¹⁹, R²⁰, R²¹, R²², R²³, R²⁴, R²⁵, R²⁶, and R²⁷ are each plurally present, they each may be different from each other.

In the formula (8), X⁷ is a -N (R²⁴) - group, N⁻, an oxygen atom or a sulfur atom. In the formula (7), R²¹ is a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent. In the formula (7), e is a number from 0 to 2. R¹⁹ and R²⁰ in the formula (7), R²² and R²³ in the formula (8), and R²⁵, R²⁶, and R²⁷ in the formula (9), which may be different from each other, are each a hydrogen atom, a halogen atom, a cyano group, a hydroxy group, a nitro group, or a carbon atom-containing group. The carbon atom-containing group has 1 to 40 carbon atoms and is an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent. R²⁴ described above is a hydrogen atom, a hydrocarbon group optionally having a substituent, or a heterocyclic group optionally having a substituent. However, at least one of R¹⁹ and R²⁰ described above is a group other than a hydrogen atom, at least one of R²² and R²³ described above is a group other than a hydrogen atom, and at least one of R²⁶ and R²⁷ described above is a group other than a hydrogen atom. In the formula (7), when a plurality of R²¹s adjoin each other, adjoining R²¹s may together form a ring. When R²¹ and one of R¹⁹ and R²⁰ adjoin each other, R²¹ and the one of R¹⁹ and R²⁰ may together form a ring. In the formula (9), R²⁵ and R²⁶ may together form a ring.

The definition of R²¹ is the same as the definition of R² in the formula (3), and specific examples and preferred examples of R²¹ are also the same as those of R². The definition of R²⁵ is the same as the definition of R⁷ in the formula (4), and specific examples and preferred examples of R²⁵ are also the same as those of R⁷.

The definition of X⁷ in the formula (8) is the same as the definition of X⁵ in the formula (4), and specific examples and preferred examples of X⁷ are also the same as those of X⁵.

The definition of R¹⁹ in the formula (7) is the same as the definition of R¹ in the formula (3), and the definition of R²⁰ in the formula (7) is the same as the definition of R⁵ in the formula (3). Specific examples and preferred examples of R¹⁹ and R²⁰ are also the same as those of R¹ and R⁵, respectively. The definition of R²² in the formula (8) is the same as the definition of R⁶ in the formula (4), and the definition of R²³ in the formula (8) is the same as the definition of R⁹ in the formula (4). Specific examples and preferred examples of R²² and R²³ are also the same as those of R⁶ and R⁹, respectively. The definition of R²⁶ in the formula (9) is the same as the definition of R⁶ in the formula (4), and the definition of R²⁷ in the formula (9) is the same as the definition of R⁹ in the formula (4). Specific examples and preferred examples of R²⁶ and R²⁷ are also the same as those of R⁶ and R⁹, respectively.

More preferably, L in the formula (1) described above has the structure represented by the following formula (10).

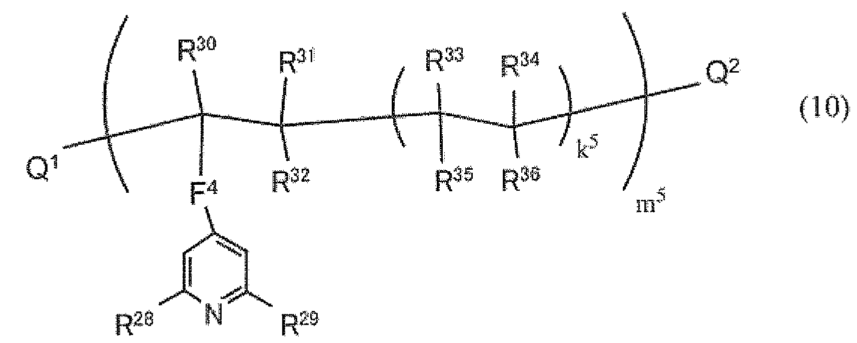

The definition of F⁴ is the same as the definition of F¹ in the formula (7), and specific examples and preferred examples of F⁴ are also the same as those of F¹. The definition of R²⁸ is the same as the definition of R¹ in the formula (3), and the definition of R²⁹ is the same as the definition of R⁵ in the formula (3). Specific examples and preferred examples of R²⁸ and R²⁹ are also the same as those of R¹ and R⁵, respectively.

A plurality of F⁴s, R²⁸s, R²⁹s, R³⁰s, R³¹s, R³²s, R³³s, R³⁴s, R³⁵s, and R³⁶s each may be different from each other.

Preferably, R³⁰, R³¹, and R³² are each a hydrogen atom, a halogen atom, a cyano group, or a carbon atom-containing group. The carbon atom-containing group has 1 to 40 carbon atoms and are an acyloxy group optionally having a substituent, a hydrocarbon oxycarbonyl group optionally having a substituent, an acyl group, a hydrocarbon group optionally having a substituent, or a hydrocarbon oxy group optionally having a substituent; more preferably a hydrogen atom, a cyano group, an acyl group, a hydrocarbon group optionally having a substituent, or a hydrocarbon oxy group optionally having a substituent; still more preferably a hydrogen atom or a hydrocarbon group optionally having a substituent; and particularly preferably a hydrogen atom. Specific examples of each of R³⁰, R³¹ and R³² and preferred examples of each of R³⁰, R³¹ and R³² are the same as those of the corresponding group described above for the compounds defined by the formulae (3) and (4).

When R³³, R³⁴, R³⁵, and R³⁶ are each a carbon atom-containing group, they are each a group having 1 to 40 carbon atoms. Preferably, at least one of R³³, R³⁴, R³⁵, and R³⁶ is a group other than a hydrogen atom. R³³, R³⁴, R³⁵, and R³⁶ are each a hydrogen atom, a halogen atom, a cyano group, an acyloxy group optionally having a substituent, a hydrocarbon oxycarbonyl group optionally having a substituent, an acyl group, a hydrocarbon group optionally having a substituent, or a hydrocarbon oxy group optionally having a substituent; more preferably a hydrogen atom, a cyano group, an acyl group, a hydrocarbon group optionally having a substituent, or a hydrocarbon oxy group optionally having a substituent; and still more preferably a hydrogen atom or a hydrocarbon group optionally having a substituent. Specific examples and preferred examples of each of the above groups are the same as those of the corresponding group described above for the compounds defined by the formulae (3) and (4).
When R³³, R³⁴, R³⁵, and R³⁶ are each a carbon atom-containing group, they each have preferably 1 to 30 carbon atoms, more preferably 1 to 20 carbon atoms, still more preferably 1 to 10 carbon atoms, and particularly preferably 1 to 6 carbon atoms. Particularly, when R³³, R³⁴, R³⁵, and R³⁶ are each a group having an aromatic ring, they each have preferably 5 to 30 carbon atoms, more preferably 6 to 20 carbon atoms, and still more preferably 6 to 10 carbon atoms.

Next, examples of the ligand L represented by the formula (2) are shown as L1 to L15. The above-described examples A1 to A52 of A correspond also to examples of the ligand L in which, in the formula (2), m¹ = 1, k¹ = 0, and Q¹ and Q² are each a hydrogen atom. p¹ to p⁸ are each a number from 0 to 10, and q¹ to q⁷ are each a number from 0 to 500.

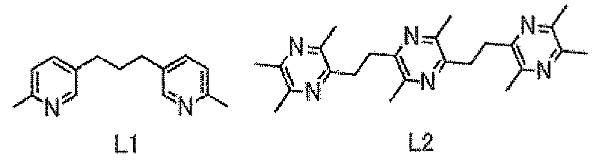

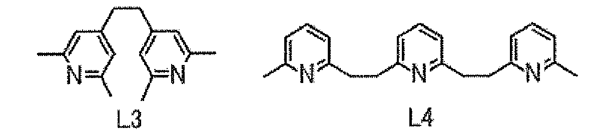

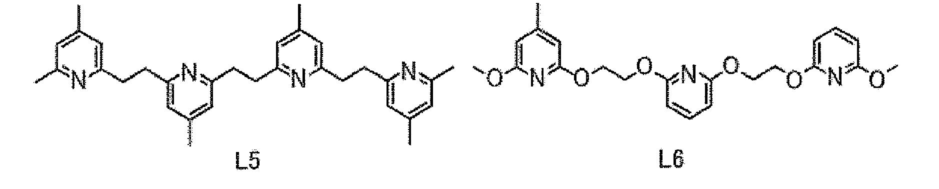

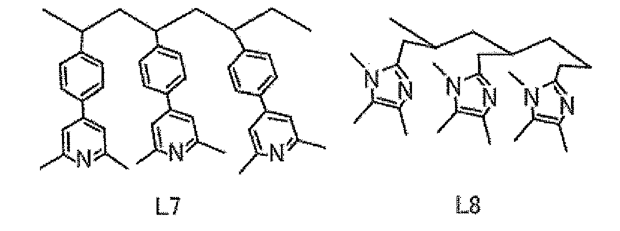

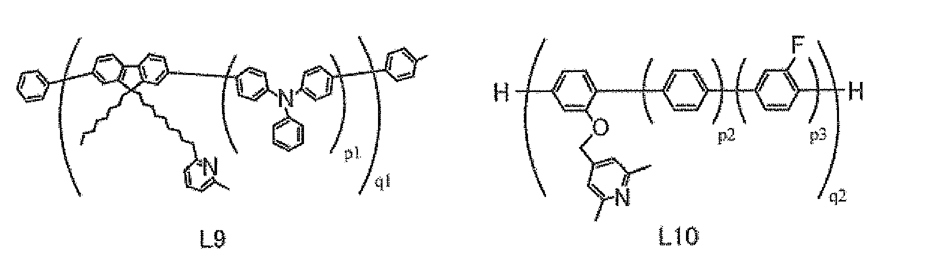

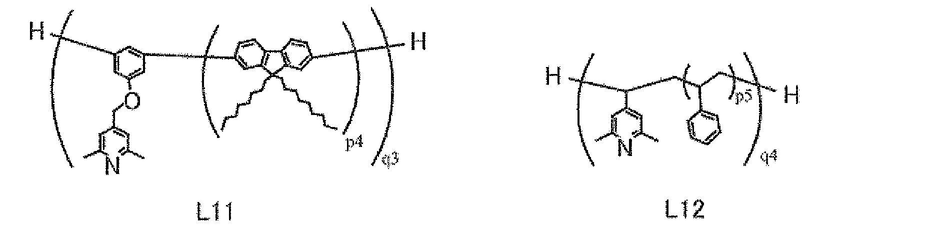

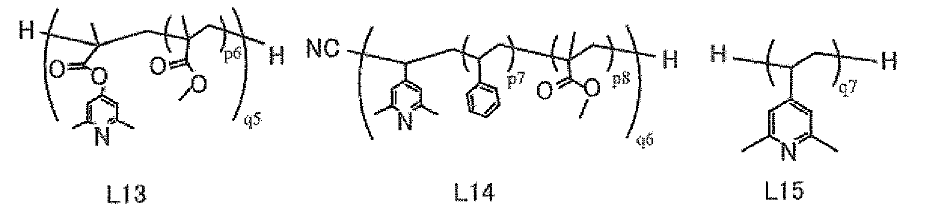

The present invention relates to a ligand represented by the following formula (11).

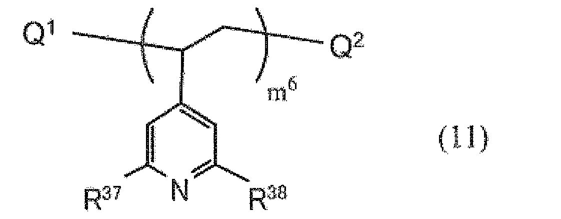

In the formula, m⁶ is a number from 2 to 10,000, preferably a number from 10 to 1,000, more preferably a number from 20 to 500, and still more preferably a number from 20 to 200. R³⁷ and R³⁸, which may be different from each other, are each a hydrocarbon group having 1 to 20 carbon atoms and optionally having a substituent. The hydrocarbon group has preferably 1 to 10 carbon atoms, more preferably 1 to 6 carbon atoms, and still more preferably 1 to 4 carbon atoms. Examples of the hydrocarbon group optionally having a substituent may include a methyl group, an ethyl group, a 1-propyl group, a 2-propyl group, a 1-butyl group, a 2-butyl group, a sec-butyl group, a tert-butyl group, a pentyl group, a hexyl group, an octyl group, and a 2-ethylhexyl group. The hydrocarbon group optionally having a substituent is preferably a methyl group, an ethyl group, a 1-propyl group, a 1-butyl group, a pentyl group, a hexyl group, an octyl group, or a 2-ethylhexyl group, more preferably a methyl group or an ethyl group, and still more preferably a methyl group.

The present invention relates to a metal coordination compound represented by the following formula (12) in which L in the following formula (12) is a ligand represented by the formula (11).

MLₐX_{b} (12)

In the formula (12), M is a metal ion, L is the ligand represented by the formula (11), X is an anion, a is a number satisfying 0 < a ≤ 6, and b is a number of 0 or more.

The metal species of the metal ion represented by M is selected from among transition metals and rare-earth metals. Examples of the metal species may include gold, silver, copper, ruthenium, rhodium, palladium, osmium, iridium, platinum, scandium, titanium, vanadium, chromium, manganese, iron, cobalt, nickel, zinc, yttrium, zirconium, niobium, molybdenum, cadmium, indium, lanthanum, cerium, praseodymium, neodymium, promethium, samarium, europium, gadolinium, terbium, dysprosium, holmium, erbium, thulium, ytterbium, lutetium, hafnium, tantalum, tungsten, rhenium, mercury, thallium, lead, and bismuth. The metal species is preferably gold, silver, copper, ruthenium, rhodium, palladium, osmium, iridium, platinum, zinc, samarium, europium, terbium, tungsten, or rhenium, more preferably gold, silver, and copper, still more preferred examples include silver or copper, and particularly preferably copper.

A metal coordination compound preferably applicable to the organic electroluminescent device of the present invention can be produced by, for example, the production method disclosed in V. H. D. Ahna et al., Zeitschrift fuer Anorganische und Allgemeine Chemie 387, 61-71 (1972). More specifically, the metal coordination compound can be synthesized by allowing a corresponding metal salt and a ligand to react in a solution.

During the reaction, an organic solvent may be used to dissolve the raw materials uniformly or to facilitate stirring if the viscosity during the reaction is high. In light of the solubilities of the reaction raw materials and the product to be obtained, the boiling point suitable for the reaction conditions, and the economical efficiency of the reaction, examples of the organic solvent used for the reaction may include: chlorine-based solvents such as chloroform, methylene chloride, 1,2-dichloroethane, 1, 1, 2-trichloroethane, chlorobenzene, and o-dichlorobenzene; ether-based solvents such as tetrahydrofuran and dioxane; aromatic hydrocarbon-based solvents such as toluene and xylene; aliphatic hydrocarbon-based solvents such as cyclohexane, methylcyclohexane, pentane, hexane, heptane, octane, nonane, and decane; ketone-based solvents such as acetone, methyl ethyl ketone, and cyclohexanone; ester-based solvents such as ethyl acetate, butyl acetate, and ethyl cellosolve acetate; polyalcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvents such as dimethylsulfoxide; and amide-based solvents such as N-methyl-2-pyrrolidone and N, N-dimethylformamide. These organic solvents may be used alone or in combination of two or more.

When m¹ is 2 or more in the formula (2), the metal coordination compound may contain trialkylphosphine, trialkylamine, and the like as an additive for improving the luminous efficiency.

Next, the organic electroluminescent device of the present invention will be described. The organic electroluminescent device of the present invention is an organic electroluminescent device in which an organic layer containing a metal coordination compound represented by the formula (1) is sandwiched between a pair of electrodes composed of an anode and a cathode. It is sufficient that the layer placed between the pair of electrodes includes at least one organic layer containing an organic compound, and at least one organic layer is generally a luminescent layer.

Examples of the organic electroluminescent device of the present invention may include an organic electroluminescent device having a single-layer type luminescent layer (anode/luminescent layer/cathode) and an organic electroluminescent device of the multi-layer type. In the organic electroluminescent device of the single-layer type, the luminescent layer contains the metal coordination compound described above. Examples of the layer structure of the organic electroluminescent device of the multi-layer type may include:
(a) anode/hole injection layer/(hole transport layer)/luminescent layer/cathode;
(b) anode/luminescent layer/electron injection layer/(electron transport layer)/cathode; and
(c) anode/hole injection layer (hole transport layer)/luminescent layer/electron injection layer/(electron transport layer)/cathode.

In (a) to (c) above, the "(hole transport layer)" and "(electron transport layer)" represent that these layer are not necessarily present at the indicated positions.

The anode of the organic electroluminescent device of the present invention supplies holes to the hole injection layer, the hole transport layer, the luminescent layer, and the like, and it is effective that an anode material has a work function of 4.5 eV or more. For the anode material, a metal, an alloy, a metal oxide, an electrically conductive compound, a mixture thereof, or the like can be used. Examples of the anode material may include: conductive metal oxides such as tin oxide, zinc oxide, indium oxide, and indium-tin oxide (ITO); metals such as gold, silver, chromium, and nickel; mixtures of these conductive metal oxides and metals; inorganic conductive materials such as copper iodide and copper sulfide; organic conductive materials such as polyanilines, polythiophenes (e.g., poly(3,4)ethylenedioxythiophene), and polypyrrole; and combinations thereof with ITO.

The cathode of the organic electroluminescent device of the present invention supplies electrons to the electron injection layer, the electron transport layer, the luminescent layer, and the like. For a cathode material, a metal, an alloy, a metal halide, a metal oxide, an electrically conductive compound, or a mixture thereof can be used. Examples of the cathode material may include: alkali metals such as Li, Na, K, and Cs, and fluorides and oxides thereof; alkaline earth metals such as Mg, Ca, and Ba, and fluorides and oxides thereof; gold, silver, lead, aluminum and alloys thereof; metal mixtures such as a sodium-potassium alloy, a metal mixture of sodium and potassium, a lithium-aluminum alloy, a metal mixture of lithium and aluminum, a magnesium-silver alloy, and a metal mixture of magnesium and silver; rare-earth metals such as ytterbium; and indium.

It is sufficient that the hole injection layer of the organic electroluminescent device of the present invention has a function of injecting holes from the anode. It is sufficient that the hole transport layer has a function of transporting holes injected from the anode, or a function as a barrier to electrons injected from the cathode. Any known materials can be appropriately selected and used for the materials of the hole injection layer and hole transport layer. Examples of such materials may include carbazole derivatives, triazole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidyne-based compounds, porphyrin-based compounds, polysilane-based compounds, poly (N-vinylcarbazole) derivatives, organic silane derivatives, metal coordination compounds having the structures described above, polymers including the above materials, aniline-based copolymers, and conductive high-molecular oligomers such as thiophene oligomers and polythiophenes. These materials may be single-component materials or compositions composed of a plurality of components. Each of the hole injection layer and hole transport layer may have a single-layer structure composed of one or two or more of these materials, or may have a multi-layer structure composed of a plurality of layers having the same composition or different compositions.

It is sufficient that the electron injection layer of the organic electroluminescent device of the present invention has a function of injecting electrons from the cathode. It is sufficient that the electron transport layer has a function of transporting electrons injected from the cathode, or a function as a barrier to holes injected from the anode. Any known materials can be appropriately selected and used for the materials of the electron injection layer and electron transport layer. Examples of such materials may include triazole derivatives; oxazole derivatives; oxadiazole derivatives; imidazole derivatives; fluorenone derivatives; anthraquinodimethane derivatives; anthrone derivatives; diphenylquinone derivatives; thiopyran dioxide derivatives; carbodiimide derivatives; fluorenylidenemethane derivatives; distyrylpyrazine derivatives; tetracarboxylic anhydrides of aromatic ring such as naphthalene and perylene; phthalocyanine derivatives; various metal coordination compounds typified by metal coordination compounds of 8-quinolinol derivatives and metal coordination compounds containing metal phthalocyanines, benzoxazole, or benzothiazole as their ligands; organic silane derivatives; and the metal coordination compounds having the structures described above. Each of the electron injection layer and electron transport layer may have a single-layer structure composed of one or two or more of these materials, or may have a multi-layer structure composed of a plurality of layers having the same composition or different compositions.

In the organic electroluminescent device of the present invention, an insulating or semiconducting inorganic compound may be used as the materials of the electron injection layer and electron transport layer.
When the electron injection layer or the electron transport layer is formed of an insulator or a semiconductor, the leakage of current can be effectively prevented, and thereby the electron injection properties can be improved. For such the insulator, at least one metal compound selected from the group consisting of alkali metal chalcogenides, alkaline earth metal chalcogenides, halides of alkali metals, and halides of alkaline earth metals may be used. Preferred examples of the alkaline earth metal chalcogenides may include CaO, BaO, SrO, BeO, BaS, and CaSe. Examples of the semiconductors for the electron injection layer or the electron transport layer may include an oxide, a nitride, an oxynitride, and the like containing at least one element selected from the group consisting of Ba, Ca, Sr, Yb, Al, Ga, In, Li, Na, Cd, Mg, Si, Ta, Sb, and Zn.
The above oxide, nitride, oxynitride, and the like may be used alone or in combination of two or more thereof.

In the present invention, a reducing dopant may be added to an interface region between the cathode and a layer adjacent thereto. Preferably, the reducing dopant is an alkali metal, an oxide of alkaline earth metal, an alkaline earth metal, a rare earth metal, an oxide of alkali metal, a halide of alkali metal, an oxide of alkaline earth metal, a halide of alkaline earth metal, an oxide of rare earth metal, a halide of rare earth metal, an alkali metal coordination compound, an alkaline earth metal coordination compound, or a rare earth metal coordination compound.

The luminescent layer of the organic electroluminescent device of the present invention has a function of injecting holes from the adjacent layer on the anode side and injecting electrons from the adjacent layer on the cathode side when an electric field is applied, a function of moving the injected charges (electrons and holes) by the force of the electric field, and a function of providing recombination sites for the electrons and holes to obtain luminescence. The luminescent layer of the organic electroluminescent device of the present invention preferably contains the metal coordination compound described above, and may also contain a host material for the metal coordination compound serving as a guest material.

Examples of the host material may include compounds having a fluorene skeleton, compounds having a carbazole skeleton, compounds having a diarylamine skeleton, compounds having a pyridine skeleton, compounds having a pyrazine skeleton, compounds having a triazine skeleton, and compounds having an arylsilane skeleton. Preferably, the T1 (the energy level of the lowest triplet excited state) of the host material is higher than the T1 of the guest material. More preferably, the difference in T1 is greater than 0.2 eV. The host material may be either a low molecular compound or a high-molecular compound. A luminescent layer in which the host material is doped with a luminescent material such as the metal coordination compound can be formed by an application of a mixture of the host material and the luminescent material, a co-vapordeposition of the host material and the luminescent material, or the like.

The amount of the metal coordination compound in a layer containing the metal coordination compound is usually 0.01 to 100 percent by weight, preferably 0.1 to 50 percent by weight, and more preferably 1 to 30 percent by weight, based on the total weight of the layer.

The above-described metal coordination compound used for the organic electroluminescent device of the present invention may be used not only for the luminescent layer but also for the hole injection layer, hole transport layer, electron injection layer, and electron transport layer.

In the organic electroluminescent device of the present invention, any known method can be used for forming each layer. Examples of the formation method may include vacuum vapor deposition methods such as a resistance heating vapor deposition method and an electron beam method, a sputtering method, an LB method, a molecular stacking method, and coating methods such as a casting method, a spin coating method, a bar coating method, a blade coating method, a roller coating method, a gravure printing method, a screen printing method, and an ink-jet method. Among these, coating methods are preferred because they contribute the simplification of production process.
When a coating method is used, the metal coordination compound is mixed with a solvent to prepare a coating solution, and the coating solution is applied to a desired layer (or an electrode) and dried to form a layer. The coating solution may contain the host material and/or a resin serving as a binder. The resin may be dissolved in the solvent or dispersed in the solvent. A non-conjugated polymer such as polyvinylcarbazole or a conjugated polymer such as a polyolefin-based polymer can be used as the resin.

Polyvinyl chloride, polycarbonate, polystyrene, polymethyl methacrylate, polybutyl methacrylate, polyester, polysulfone, polyphenylene oxide, polybutadiene, poly(N-vinylcarbazole), hydrocarbon resin, ketone resin, phenoxy resin, polyamide, ethyl cellulose, vinyl acetate, ABS resin, polyurethane, melamine resin, unsaturated polyester resin, alkyd resin, epoxy resin, silicon resin, or the like may also be used. The solution may contain an antioxidant, a viscosity modifier, or the like.

For the solvent of the solution, a known stable solvent that can dissolve or disperse components of a layer uniformly can be appropriately selected and used. Examples of such a solvent may include: alcohols such as methanol, ethanol, and isopropyl alcohol; ketones such as acetone and methyl ethyl ketone; organic chlorines such as chloroform and 1,2-dichloroethane; aromatic hydrocarbons such as benzene, toluene, and xylene; aliphatic hydrocarbons such as normal hexane and cyclohexane; amides such as dimethyl formamide; and sulfoxides such as dimethylsulfoxide. The solvent may be composed of a single component or may be a mixture of a plurality of components.

In an ink-jet method, a high-boiling point solvent such as anisole or bicyclohexylbenzene may be used as a component for preventing, for example, evaporation from nozzles. Preferably, the viscosity of the solution at 25°C is adjusted to 1 to 100 mPa·s by selecting the components.

In the organic electroluminescent device of the present invention, the preferred thickness of the organic layer varies depending on the types of materials and the layer structure. Generally, if the thickness is too small, defects such as pinholes are more likely to occur. If the thickness is too large, a high applied voltage is required, causing deterioration of efficiency. Generally, the thickness is preferably in the range of several nm to 1 µm.

The organic electroluminescent device of the present invention can be used for light sources for illumination, light sources for signs, light sources for backlights, display apparatuses, printer heads, or the like. In such display apparatuses, a driving technique such as a known driving circuit may be used, and the segment type configuration, the dot matrix type configuration, or the like can be selected.

### Examples

Examples will be shown to describe the present invention in detail.
The weight average molecular weight (Mw) and number average molecular weight (Mn) of a polymer were measured using a gel permeation chromatography (GPC) (HLC-8220GPC, product of Tosoh Corporation) and were determined as a polystyrene equivalent weight average molecular weight and a polystyrene equivalent number average molecular weight. The sample used for the measurement was dissolved in tetrahydrofuran at a concentration of about 0.5 percent by weight, and 50 µL of the solution was injected into the GPC. Tetrahydrofuran was used as the mobile phase of the GPC and was allowed to flow at a flow rate of 0.5 mL/min. The emission spectrum and emission lifetime were measured at an excitation wavelength of 350 nm using a fluorescence spectrophotometer (product name: Fluorolog-Tau3, product of JOBINYVON-SPEX). The excitation lifetime was measured at the wavelength of the luminescence peak.

### <Example 1>

### <Synthesis example of metal coordination compound>

Under an argon atmosphere, copper(1) chloride (500 mg, 5.1 mmol) was added to 2,6-dimethylpyridine (5mL), and the mixture was heated to 100°C under stirring, thus obtaining a homogeneous solution. The obtained solution was allowed to cool to room temperature, and colorless crystals were precipitated. The crystals were collected by filtration, washed with acetone, and then, vacuum-dried at 50°C for 5 hours, thus obtaining a metal coordination compound ((Cu⁺)₁(2, 6-dimethylpyridine)₁(Cl⁻)₁) (702 mg, yield: 68%) .
Elemental Analysis: Found(%) C:40.69, H:4.30, N:6.89, C1:16.50, Calcd for C₇H₉NCuC1 (%) C:40.78, H:4.40, N:6.79, Cl:17.20
The luminescence peak wavelength (excitation lifetime) of a powder sample was 446 nm (4.7 µs).

### <Production example of organic electroluminescent device>

On a glass substrate having a 150 nm-thick ITO film formed by sputtering, a poly(ethylenedioxythiophene)/polystyrene sulfonate solution (product name: Baytron P, Bayer Holding Ltd.) was applied by spin coating to form a film in a thickness of 65 nm, and then dried on a hot plate at 200°C for 10 minutes.
Next, the metal coordination compound synthesized in the above synthesis example was mixed with a polymer having repeating units F5 and F8 shown below in a molar ratio of F8:F5 = 8:2 so that the weight ratio of the metal coordination compound to the polymer is 2:8, and a film of the mixture was formed by spin coating. The thickness of the film was about 60 nm. The film formed was dried in a nitrogen gas atmosphere at 130°C for 10 minutes. Then barium of about 4 nm was vapor-deposited, and aluminum of about 100 nm was vapor-deposited to form a cathode. An organic electroluminescent device was thereby produced.
After the obtained organic electroluminescent device was exposed to the air, a voltage was applied thereto, and electroluminescence with a peak at about 500 nm was obtained.

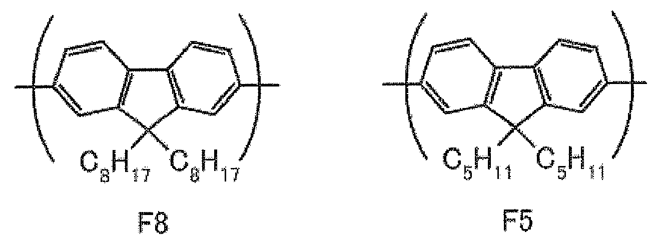

The luminescence peak wavelength of the metal coordination compound in the organic electroluminescent device was located at about 500 nm, which was on the long wavelength side of the luminescence peak for the powder. Such shift of the luminescence peak to the long wavelength side has also been observed when a powder sample of a metal coordination compound coordinated by pyridine was dissolved in a solvent, as described in the Non-Patent Document 1.

### <Example 2>

### <Synthesis example of poly(4-vinyl-2,6-dimethylpyridine)>

Poly(4-vinyl-2,6-dimethylpyridine) was synthesized according to the following scheme.

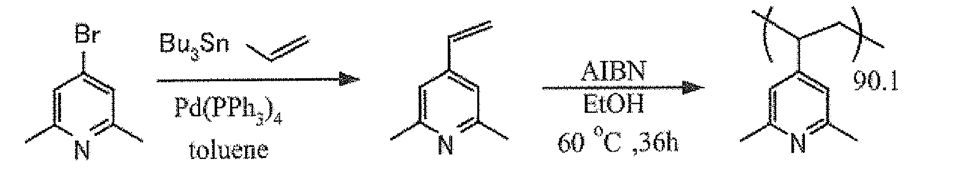

A 500 mL four-neck flask substituted with nitrogen was charged with 4-bromo-2,6-dimethylpyridine (9.80 g, 52.7 mmol, product of FOCUS), Pd(PPh₃)₄ (6.09 g, 5.27 mmol), and dehydrated toluene (260 mL), and bubbled with nitrogen for 15 minutes. Then, tributylvinyltin (20.04 g, 63.21 mmol) was added, and the temperature was increased to a reflux temperature (105 to 107°C). The mixture was held at the reflux temperature in a nitrogen atmosphere for 17 hours, cooled, and condensed using an evaporator, thus obtaining 30.29 g of a black liquid.
Petroleum ether (100 mL) was added to the obtained liquid. The mixture was filtrated through Celite, and then the solvent was evaporated. The residue was purified by silica gel column chromatography (eluent: hexane:ethyl acetate = 3:1), thus obtaining 3.0 g of 4-vinyl-2,6-dimethylpyridine (43%).
¹H NMR (400 MHz, CDCl₃) : δ 6.95 (s, 2H), 6.60 (m, 1H), 5.92 (d, J = 17.6 Hz, 1H), 5.42 (d, J = 10. Hz, 1H), 2.52 (s, 6H) .

A 50 mL two-neck flask substituted with argon was charged with the 4-vinyl-2,6-dimethylpyridine (500 mg, 3.75 mmol) and dehydrated ethanol (2 mL). After oxygen was removed by freeze-pump-thaw degassing, AIBN (azobisisobutyronitrile) (3 mg, 0.018 mmol, product of TCI) was added, and the mixture was stirred at 60°C for 24 hours. AIBN (30 mg, 0.18 mmol) was further added, and the mixture was stirred at 60°C for 15 hours. Then the reaction mixture was poured to hexane, and the precipitate formed was collected, thus obtaining 460 mg of the desired compound (92%). The number average molecular weight (Mn) of the obtained poly(4-vinyl-2,6-dimethylpyridine) was 12,000, and the weight average molecular weight (Mw) was 31,000. The distribution (Mw/Mn) was 2.6. Since the number average molecular weight of the poly(4-vinyl-2,6-dimethylpyridine) used was 12,000 and the molecular weight of the repeating units was 133.2, the polymer obtained was found to be a 90.1-me on average.

### <Synthesis example of metal coordination compound>

Under an argon atmosphere, copper(1) chloride (7.5 mg, 0.075 mmol) and poly(4-vinyl-2,6-dimethylpyridine) (100 mg, repeating unit: 0.75 mmol) were dissolved in dehydrated acetonitrile (30 mL), and the mixture was stirred at room temperature. The solvent was evaporated under reduced pressure. A metal coordination compound ((Cu⁺)₁(poly(4-vinyl-2,6-dimethylpyridine))_{0.11}(Cl⁻)₁) was thereby obtained.
The luminescence peak wavelength (excitation lifetime) of a powder sample was 480 nm (1.0 µs) .

### <Production example of organic electroluminescent device>

On a glass substrate having a 150 nm-thick ITO film formed by sputtering, a solution of poly(ethylenedioxythiophene) and polystyrene sulfonate (product name: Baytron P, Bayer Holding Ltd.) was applied by spin coating to form a film in a thickness of 65 nm, and then dried on a hot plate at 200°C for 10 minutes. Next, a composition for forming a hole transport layer was applied by spin coating to form a film in a thickness of 20 nm, and the film formed was dried on a hot plate at 190°C for 20 minutes, thus forming the hole transport layer.

The composition for forming the hole transport layer was synthesized by the following method.
A1L three-neck round bottom flask equipped with a reflux condenser and an overhead stirrer was charged with 2,7-bis(1,3,2-dioxyborole)-9,9-di(1-octyl) fluorene (3.863 g, 7.283 mmol), N,N-di(p-bromophenyl)-N-(4-(butane-2-yl)phenyl)amine (3.177 g, 6.919 mmol), and di(4-bromophenyl) benzocyclobutaneamine (156.3 mg, 0.364 mmol)).

Next, methyltrioctylammonium chloride (product name: Aliquat336 (registered trademark), product of Aldrich) (2.29 g) and then 50mL of toluene were added. After a PdCl₂(PPh₃)₂ catalyst (4.9 mg) was added, the mixture was stirred in an oil bath at 105°C for 15 minutes. An aqueous solution of sodium carbonate (2.0 M, 14 mL) was added, and the resultant mixture was stirred in an oil bath at 105°C for 16.5 hours.

Then phenylboronic acid (0.5 g) was added, and the resultant mixture was stirred for 7 hours. The aqueous phase was removed, and the organic phase was washed with 50 mL of water.

The organic phase was returned to the reaction flask, and 0.75 g of sodium diethyldithiocarbamate and 50 mL of water were added. The resultant mixture was stirred in an oil bath at 85°C for 16 hours. The aqueous phase was removed, and the organic phase was washed three times with 100 mL of water and subjected to a column of silica gel and basic alumina.

Next, the collected solution was poured to methanol to precipitate a polymer, and the obtained polymer was vacuum-dried at 60°C, thus obtaining 4.2 g of the composition for the hole transport layer. The polystyrene equivalent weight average molecular weight (Mw) of the composition for the hole transport layer was 124,000, and the distribution (Mw/Mn) was 2.8.

N,N'-dicarbazoly1-1,3-benzene was mixed with 30 percent by weight of the metal coordination compound synthesized in Example 2, and the mixture was applied by spin coating to form a film on the substrate having the hole transport layer formed thereon. The thickness of the film was 40 nm. The film formed was dried in a nitrogen gas atmosphere at 60°C for 20 minutes, thus obtaining a luminescent layer.

Then lithium fluoride of about 1 nm was vapor-deposited, and then aluminum of about 80 nm was vapor-deposited to form a cathode. An organic electroluminescent device was thereby produced.

After the obtained organic electroluminescent device was exposed to the air, a voltage was applied thereto, and electroluminescence with a peak at about 515 nm was obtained.

### <Example 3>

### <Synthesis example of metal coordination compound>

Under an argon atmosphere, copper(1) iodide (400 mg, 2.1 mmol) and 1,2,4,5-tetramethylimidazole (1 g, 8.1 mmol) were dissolved in dehydrated acetone (10 mL) under heating at 50°C and stirring, thus forming a homogeneous solution. The solution was allowed to cool to room temperature, and colorless crystals were precipitated. The crystals were collected by filtration, washed with acetone, and vacuum-dried at 50°C for 5 hours, thus obtaining a metal coordination compound ((Cu⁺)₁(1,2,4,5-tetramethylimidazole)₂(I⁻)₁) (674 mg, yield: 73%).
Elemental Analysis: Found(%) C:38.54, H:5.44, N:12.84, 1:28.17, Cu:13.3, Calcd for C₁₄H₁₂N₂CuI(%) C:38.32, H:5.51, N:12.77, 1:28.92, Cu:14.5
The luminescence peak wavelength of a powder sample was 425 nm.

### <Production example of organic electroluminescent device>

An organic electroluminescent device is produced in the same manner as in the production example of the organic electroluminescent device in Example 1. After the obtained organic electroluminescent device is exposed to the air, a voltage is applied thereto, and electroluminescence is obtained.

### <Example 4>

### <Synthesis example of metal coordination compound>

Silver(I) tetrafluoroborate (0.4 mg, 0.002 mmol) and poly(4-vinyl-2,6-dimethylpyridine) (1.9 mg, repeating unit: 0.014 mmol) were dissolved in dehydrated dichloromethane (1 mL), and the mixture was stirred at room temperature. The solvent was evaporated under reduced pressure. A metal coordination compound ((Ag⁺)₁(poly(4-vinyl-2,6-dimethylpyridine))_{0.08}(BF₄⁻)₁) was thereby obtained.
The luminescence peak wavelength (excitation lifetime) of a powder sample was 390 nm.

### <Production example of organic electroluminescent device>

An organic electroluminescent device is produced in the same manner as in the production example of the organic electroluminescent device in Example 1. After the obtained organic electroluminescent device is exposed to the air, a voltage is applied thereto, and electroluminescence is obtained.

### <Comparative Example 1>

### <Synthesis example of metal coordination compound>

Under an argon atmosphere, pyridine (5 mL) and dehydrated acetone (10 mL) were added to copper(I) iodide (400 mg, 2.1 mmol), and the mixture was stirred at room temperature, thus forming a homogeneous solution. The solution was cooled to -18°C, and colorless crystals were precipitated. The crystals were collected by filtration and vacuum-dried at 50°C for 5 hours, thus obtaining (Cu⁺)₁(pyridine)₁(I⁻)₁ (312 mg, yield: 44%).
Elemental Analysis: Found(%) C:20.89, H:1.76, N:4.82, 1:44.15, Calcd for C₅H₅NCuI(%) C:22.28, H:1.87, N:5.20, 1:47.08
The luminescence peak wavelength (excitation life) of a powder sample was 560 nm (6.3 µs).

### <Production example of comparative organic

### electroluminescent device>

An organic electroluminescent device (comparative organic electroluminescent device) was produced in the same manner as in the production example of the organic electroluminescent device in Example 1 except that (Cu)₁(pyridine)₁(I)₁ was used as the metal coordination compound. After the obtained comparative organic electroluminescent device was exposed to the air, a voltage is applied thereto. However, no electroluminescence was observed.

### <Comparative Example 2>

A comparative compound was obtained in the same manner as in the synthesis example of the metal coordination compound in Example 2 except that poly(4-vinyl pyridine) (Aldrich, average Mw: ∼60,000) was used instead of the poly(4-vinyl-2,6-dimethylpyridine) used in the synthesis example of the metal coordination compound in Example 2. The obtained comparative compound rapidly changed its color to black during air exposure and was decomposed. As the compound was decomposed, the luminescence by ultraviolet excitation (excitation wavelength: 365 nm) disappeared. Therefore, when a device is produced in the same manner as in the production example of the organic electroluminescent device in Example 2, and the obtained organic electroluminescent device is exposed to the air, no electroluminescence is observed even when a voltage is applied.

### <Comparative Example 3>

Comparative compound 3 was obtained in the same manner as in synthesis example 2 of the metal coordination compound except that imidazole was used instead of 1,2,4,5-tetramethylimidazole used in the synthesis example of the metal coordination compound in Example 3. The obtained comparative compound 3 rapidly changed its color to black during air exposure and was decomposed. As the compound was decomposed, the luminescence by ultraviolet excitation (excitation wavelength: 365 nm) disappeared. Therefore, when a device is produced in the same manner as in the production example of the organic electroluminescent device in Example 1, and the obtained organic electroluminescent device is exposed to the air, no electroluminescence is observed even when a voltage is applied.

As is clear from the Examples above, the organic electroluminescent devices of the present invention that contain the metal coordination compounds including 2,6-dimethylpyridine, poly(4-vinyl-2,6-dimethylpyridine), and 1,2,4,5-tetramethylimidazole as ligands have an electroluminescent function even after air exposure. Therefore, it can be recognized that the organic electroluminescent devices of the present invention have sufficient durability against, in particular, oxygen in the air.

In the metal coordination compound obtained in Comparative Example 1 in which pyridine was used as ligands, the metal coordination compound obtained in Comparative Example 2 in which poly(4-vinylpyridine) was used as ligands, and the metal coordination compound obtained in Comparative Example 3 in which imidazole was used as ligands, during air exposure, rapid change in color and rapid decomposition resulting in the disappearance of ultraviolet excited luminescence were found. Since these metal coordination compounds are easily decomposed during device production or subsequent air exposure, electroluminescent devices cannot be obtained. As shown in Comparative Example 2, when the organic electroluminescent device containing the metal coordination compound including pyridine as ligands was exposed to the air, no electroluminescence was observed. Therefore, it can be recognized that the organic electroluminescent device in the above Comparative Example does not have sufficient durability against oxygen.

## Claims

1. An organic electroluminescent device in which an organic layer containing a metal coordination compound represented by the following formula (1) is sandwiched between a pair of electrodes composed of an anode and a cathode,
MLₐX_{b} (1)
wherein
M is an ion of a metal of Group 11 of the Periodic Table,
L is a ligand represented by the formula (2) shown below,
X is an anion,
a is a number satisfying 0 < a ≤ 6, and
b is a number of 0 or more, 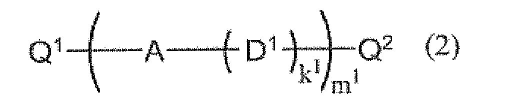
wherein
A is a divalent group formed by removing two hydrogen atoms from the formula (3) or the formula (4) shown below,
D¹ is a divalent group having 1 to 50 carbon atoms,
k¹ is a number of 0 or more,
m¹ is a number of 1 or more,
when at least one of A and D¹ is plurally present, the at least one of A and D¹ that is plurally present may be different from each other, and
Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, 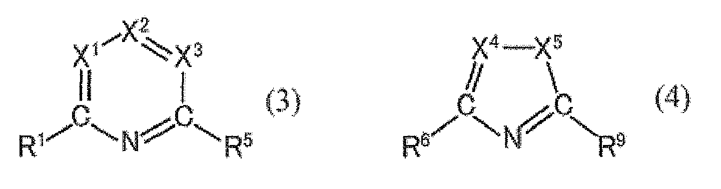
wherein
x¹ is a nitrogen atom or a =C(R²)- group, X² is a nitrogen atom or a -C(R³) = group, X³ is a nitrogen atom or a =C (R⁴) - group, X⁴ is a nitrogen atom or a =C(R⁷)- group, X⁵ is a -N(R⁸) - group, N⁻, an oxygen atom, or a sulfur atom, and at least one of X¹, X², and X³ is a =C(R²) - group, a - C (R³) = group, or a =C(R⁴) - group,
R¹, R², R³, R⁴, R⁵, R⁶, R⁷ and R⁹, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, and R⁸ is a hydrogen atom, a hydrocarbon group optionally having a substituent, or a heterocyclic group optionally having a substituent, provided that at least one of R¹ and R⁵ is a group other than a hydrogen atom and that at least one of R⁶ and R⁹ is a group other than a hydrogen atom, and
each of a combination of R¹ and R², a combination of R² and R³, a combination of R³ and R⁴, a combination of R⁴ and R⁵, a combination of R⁶ and R⁷, a combination of R⁷ and R⁸, and a combination of R⁸ and R⁹ may together form a ring.

2. The organic electroluminescent device according to claim 1, wherein, in the formulae (3) and (4), when R¹ to R⁹ are each a group other than a hydrogen atom, R¹ to R⁹ are each a hydrocarbon group optionally having a substituent.

3. The organic electroluminescent device according to claim 1, wherein, in the formula (2), A is a divalent group of the following formula (5) or (6) with two hydrogen atoms removed therefrom, 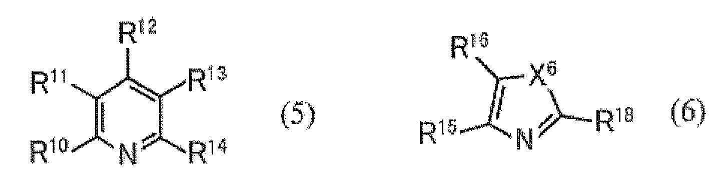 wherein
X⁶ is a -N(R¹⁷)- group, N⁻, an oxygen atom, or a sulfur atom,
R¹⁰, R¹¹, R¹², R¹³, R¹⁴, R¹⁵, R¹⁶ and R¹⁸, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, and R¹⁷ is a hydrogen atom, a hydrocarbon group optionally having a substituent, or a heterocyclic group optionally having a substituent, provided that at least one of R¹⁰ and R¹⁴ is a group other than a hydrogen atom and that at least one of R¹⁵ and R¹⁸ is a group other than a hydrogen atom, and
each of a combination of R¹⁰ and R¹¹, a combination of R¹¹ and R¹², a combination of R¹² and R¹³, a combination of R¹³ and R¹⁴, a combination of R¹⁵ and R¹⁶, a combination of R¹⁶ and R¹⁷, and a combination of R¹⁷ and R¹⁸ may together form a ring.

4. The organic electroluminescent device according to claim 1, wherein, in the formula (2), when A is plurally present, the number of covalent bonds connecting any two coordinating nitrogen atoms by a shortest path is 5 or more.

5. The organic electroluminescent device according to claim 1, wherein, in the formula (2), m¹ is a number from 2 to 10,000.

6. The organic electroluminescent device according to claim 1, wherein, in the metal coordination compound represented by the formula (1), L is a ligand represented by the following formula (7), 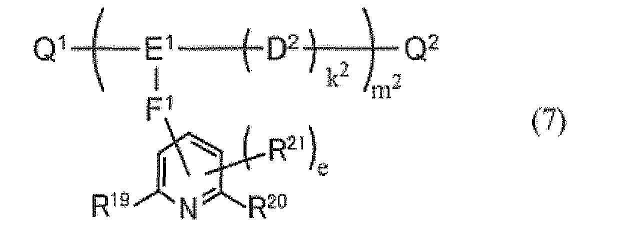 wherein
Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
D² is a divalent group having 1 to 50 carbon atoms, E¹ is a trivalent group having 1 to 50 carbon atoms, F¹ is a direct bond or a divalent group having 1 to 20 carbon atoms,
m² is a number of 2 or more,
k² is a number of 0 or more,
when E¹, F¹, D², R¹⁹, R²⁰, and R²¹ are each plurally present, they each may be different from each other,
R²¹ is a halogen atom, a cyano group, a nitro group, an acyloxy group optionally having a substituent, a hydrocarbon oxycarbonyl group optionally having a substituent, a carboxylate group, an acylamido group, an imino group, a substituted silyl group, a hydroxy group, an acyl group, a hydrocarbon group optionally having a substituent, a hydrocarbon oxy group optionally having a substituent, a hydrocarbon thio group optionally having a substituent, or a heterocyclic group optionally having a substituent,
e is a number from 0 to 2,
R¹⁹ and R²⁰, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, provided that at least one of R¹⁹ and R²⁰ is a group other than a hydrogen atom,
when a plurality of R²¹s adjoin each other, adjoining R²¹s may together form a ring, and
when R²¹ and one of R¹⁹ and R²⁰ adjoin each other, R²¹ and the one of R¹⁹ and R²⁰ may together form a ring.

7. The organic electroluminescent device according to claim 1, wherein, in the metal coordination compound represented by the formula (1), L is a ligand represented by the following formula (8), 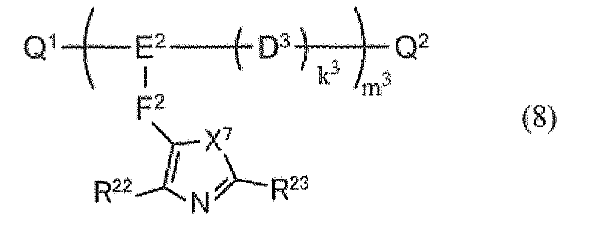 wherein
Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
D³ is a divalent group having 1 to 50 carbon atoms,
E² is a trivalent group having 1 to 50 carbon atoms,
F² is a direct bond or a divalent group having 1 to 20 carbon atoms,
m³ is a number of 2 or more,
k³ is a number of 0 or more,
when E², F², D³, R²², R²³, and R²⁴ are each plurally present, they each may be different from each other,
X⁷ is a -N(R²⁴)- group, N⁻, an oxygen atom, or a sulfur atom,
R²² and R²³, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, and R²⁴ is a hydrogen atom, a hydrocarbon group optionally having a substituent, or a heterocyclic group optionally having a substituent, provided that at least one of R²² and R²³ is a group other than a hydrogen atom.

8. The organic electroluminescent device according to claim 1, wherein, in the metal coordination compound represented by the formula (1), L is a ligand represented by the following formula (9), 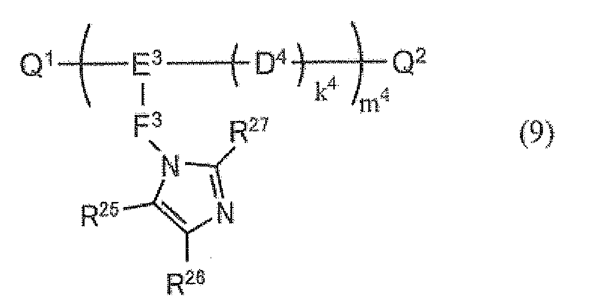 wherein
Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
D⁴ is a divalent group having 1 to 50 carbon atoms,
E³ is a trivalent group having 1 to 50 carbon atoms,
F³ is a direct bond or a divalent group having 1 to 20 carbon atoms,
m⁴ is a number of 2 or more,
k⁴ is a number of 0 or more,
when E³, F³, D⁴, R²⁵, R²⁶, and R²⁷ are each plurally present, they each may be different from each other,
R²⁵, R²⁶ and R²⁷, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, provided that at least one of R²⁶ and R²⁷ is a group other than a hydrogen atom, and
R²⁵ and R²⁶ may together form a ring.

9. The organic electroluminescent device according to claim 1, wherein, in the metal coordination compound represented by the formula (1), L is a ligand represented by the following formula (10), 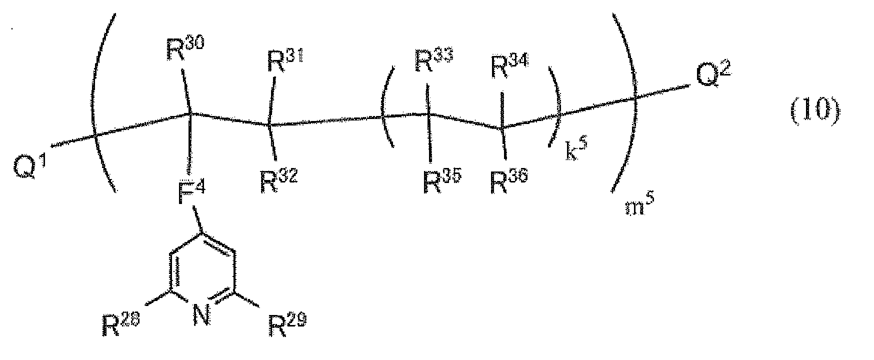 wherein
Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
F⁴ is a direct bond or a divalent bond having 1 to 20 carbon atoms,
m⁵ is a number of 2 or more,
k⁵ is a number of 0 or more,
when F⁴, R²⁸, R²⁹, R³⁰, R³¹, R³², R³³, R³⁴, R³⁵, and R³⁶ are each plurally present, they each may be different from each other,
R²⁸ and R²⁹, which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent, provided that at least one of R²⁸ and R²⁹ is a group other than a hydrogen atom, and
R³⁰, R³¹, R³², R³³, R³⁴, R³⁵, and R³⁶, which may be different from each other, are each a hydrogen atom, a halogen atom, a cyano group, an acyloxy group optionally having a substituent, a hydrocarbon oxycarbonyl group optionally having a substituent, an acylamido group, a substituted silyl group, a hydroxy group, an acyl group, a hydrocarbon group optionally having a substituent, a hydrocarbon oxy group optionally having a substituent, or a hydrocarbon thio group optionally having a substituent.

10. The organic electroluminescent device according to claim 9, wherein, in the ligand represented by the formula (10), when any of R²⁸ R²⁹, R³⁰, R³¹, R³², R³³, R³⁴, R³⁵, and R³⁶ is a carbon atom-containing group, the carbon atom-containing group has 1 to 40 carbon atoms.

11. The organic electroluminescent device according to claim 1, wherein, in the metal coordination compound represented by the formula (1), L is a ligand selected from among 2,6-dimethylpyridine; poly(4-vinyl-2,6-dimethylpyridine) having a number average molecular weight of at most 13,000; and 1,2,4,5-tetramethylimidazole.

12. The organic electroluminescent device according to claim 1, wherein, in the metal coordination compound represented by the formula (1), M is a monovalent copper ion or a monovalent silver ion.

13. A ligand represented by the following formula (11), 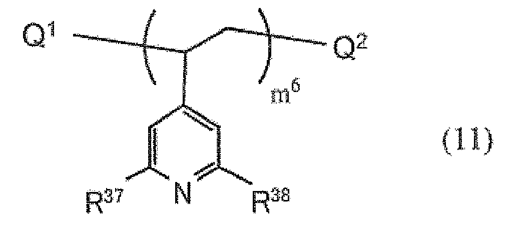 wherein
Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
m⁶ is a number from 2 to 10,000, and
R³⁷ and R³⁸, which may be different from each other and may have a substituent, are each a hydrocarbon group having 1 to 20 carbon atoms.

14. A metal coordination compound represented by the following formula (12),
MLₐX_{b} (12)
wherein
M is a metal ion,
L is a ligand represented by the formula (11) shown below,
X is an anion, a is a number satisfying 0 < a ≤ 6, and
b is a number of 0 or more, 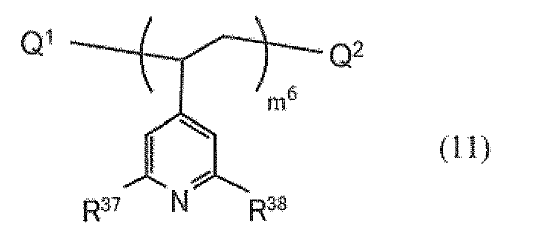
wherein
Q¹ and Q², which may be different from each other, are each a hydrogen atom, a halogen atom, a cyano group, a hydroxy group, a nitro group, an acyloxy group optionally having a substituent, a hydrocarbon oxycarbonyl group optionally having a substituent, a carboxylate group, an acylamido group, an imino group, a substituted silyl group, an acyl group, a hydrocarbon group optionally having a substituent, a hydrocarbon oxy group optionally having a substituent, a hydrocarbon thio group optionally having a substituent, or a heterocyclic group optionally having a substituent,
m⁶ is a number from 2 to 10,000, and
R³⁷ and R³⁸, which may be different from each other and have a substituent, are each a hydrocarbon group having 1 to 20 carbon atoms.
